(19) Europäisches Patentamt
European Patent Office
Office européen des brevets

(11) EP 0 804 421 B1

# (12) EUROPÄISCHE PATENTSCHRIFT

(45) Veröffentlichungstag und Bekanntmachung des Hinweises auf die Patenterteilung:
**16.09.1998 Patentblatt 1998/38**

(21) Anmeldenummer: **95924888.1**

(22) Anmeldetag: **21.06.1995**

(51) Int Cl.[6]: **C07D 231/22**, A01N 43/56, C07D 231/20, C07D 401/04, C07D 403/04, C07C 239/10, C07C 271/28, C07C 271/58

(86) Internationale Anmeldenummer:
**PCT/EP95/02396**

(87) Internationale Veröffentlichungsnummer:
**WO 96/01256 (18.01.1996 Gazette 1996/04)**

## (54) 2- (DIHYDRO)PYRAZOLYL-3'-OXYMETHYLEN]-ANILIDE ALS SCHÄDLINGSBEKÄMPFUNGSMITTEL UND FUNGIZIDE

USE OF 2- (DIHYDRO)PYRAZOLYL-3'-OXYMETHYLENE]-ANILIDES AS PEST-CONTROL AGENTS AND FUNGICIDES

2- (DIHYDRO)PYRAZOLYL-3'-OXYMETHYLENE]-ANILIDES UTILISES COMME PESTICIDES ET COMME FONGICIDES

(84) Benannte Vertragsstaaten:
**AT BE CH DE DK ES FR GB GR IE IT LI NL PT SE**

(30) Priorität: **06.07.1994 DE 4423612**

(43) Veröffentlichungstag der Anmeldung:
**05.11.1997 Patentblatt 1997/45**

(73) Patentinhaber: **BASF AKTIENGESELLSCHAFT**
**67056 Ludwigshafen (DE)**

(72) Erfinder:
- **MÜLLER, Bernd**
  **D-67227 Frankenthal (DE)**
- **KÖNIG, Hartmann**
  **D-69115 Heidelberg (DE)**
- **KIRSTGEN, Reinhard**
  **D-67434 Neustadt (DE)**
- **OBERDORF, Klaus**
  **D-69117 Heidelberg (DE)**
- **RÖHL, Franz**
  **D-67105 Schifferstadt (DE)**
- **GÖTZ, Norbert**
  **D-67547 Worms (DE)**
- **SAUTER, Hubert**
  **D-68167 Mannheim (DE)**
- **LORENZ, Gisela**
  **D-67434 Hambach (DE)**
- **AMMERMANN, Eberhard**
  **D-64646 Heppenheim (DE)**

(56) Entgegenhaltungen:
**WO-A-93/15046**

- **JOURNAL OF THE CHEMICAL SOCIETY, PERKIN TRANSACTIONS 2, Nr.12, 1981, LETCHWORTH GB Seiten 1596 - 1598 T. SONE ET AL. 'Kinetics and Mechanisms of the Bamberger Rearrangement. Part 4. Rearrangements of Sterically Hindered Phenylhydroxylamines to 4-Aminophenols in Aqueous Sulphuric Acid Solution'**

Bemerkungen:
Die Akte enthält technische Angaben, die nach dem Eingang der Anmeldung eingereicht wurden und die nicht in dieser Patentschrift enthalten sind.

EP 0 804 421 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft 2-[(Dihydro)pyrazolyl-3'-oxymethylen]-anilide der Formel I

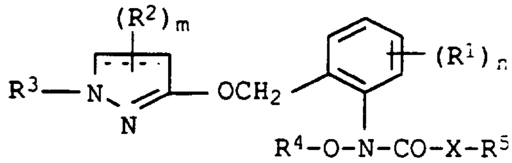

I

in der ... für eine Einfach- oder Doppelbindung steht und die Indices und die Substituenten die folgende Bedeutung haben:

n 0, 1, 2, 3 oder 4, wobei die Substituenten $R^1$ verschieden sein können, wenn n größer als 1 ist;

m 0, 1 oder 2, wobei die Substituenten $R^2$ verschieden sein können, wenn m größer als 1 ist;

X eine direkte Bindung, O oder $NR^a$;

$R^a$ Wasserstoff, Alkyl, Alkenyl, Alkinyl, Cycloalkyl oder Cycloalkenyl;

$R^1$ Nitro, Cyano, Halogen,

ggf. subst. Alkyl, Alkenyl, Alkinyl, Alkoxy, Alkenyloxy, Alkinyloxy oder

für den Fall, daß n für 2 steht zusätzlich eine an zwei benachbarte Ringatome gebundene ggf. subst. Brücke, welche drei bis vier Glieder aus der Gruppe 3 oder 4 Kohlenstoffatome, 2 bis 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann;

$R^2$ Nitro, Cyano, Halogen, $C_1$-$C_4$-Alkyl, $C_1$-$C_4$-Halogenalkyl, $C_1$-$C_4$-Alkoxy, $C_1$-$C_4$-Alkylthio oder $C_1$-$C_4$-Alkoxycarbonyl;

$R^3$ ggf. subst. Alkyl, Alkenyl oder Alkinyl;

ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, oder

ein ggf. subst. ein- oder zweikerniger aromatischer Rest, welcher neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann;

$R^4$ Wasserstoff,
ggf. subst. Alkyl, Alkenyl, Alkinyl, Cycloalkyl, Cycloalkenyl, Alkylcarbonyl oder Alkoxycarbonyl;

$R^5$ Alkyl, Alkenyl, Alkinyl, Cycloalkyl oder Cycloalkenyl, oder
für den Fall, daß X für $NR^a$ steht, zusätzlich Wasserstoff.

Außerdem betrifft die Erfindung Verfahren und Zwischenprodukte zur Herstellung dieser Verbindungen, sie enthaltende Mittel sowie ihre Verwendung zur Bekämpfung von tierischen Schädlingen oder Schadpilzen.

Aus der WO-A 93/15,046 sind 2-[Pyrazolyl-4-oxymethylen]-anilide zur Bekämpfung von tierischen Schädlingen und Schadpilzen bekannt.

Der vorliegenden Erfindung lagen Verbindungen mit verbesserter Wirkung als Aufgabe zugrunde.

Demgemäß wurden die eingangs definierten Verbindungen gefunden. Desweiteren wurden Verfahren und Zwischenprodukte zu ihrer Herstellung, sie enthaltende Mischungen sowie Verfahren zur Bekämpfung von tierischen Schädlingen und Schadpilzen unter Verwendung der Verbindungen I gefunden.

Die Verbindungen I sind auf verschiedenen Wegen erhältlich.

Man erhält diejenigen Verbindungen I, in denen R4 Wasserstoff bedeutet, und X für eine direkte Bindung oder Sauerstoff steht, beispielsweise dadurch, daß man ein Benzylderivat der Formel II in Gegenwart einer Base mit einem 3-Hydroxy(dihydro)pyrazol der Formel III in das entsprechende 2-[(Dihydro)pyrazolyl-3-oxymethylen]-nitrobenzol der Formel IV überführt, IV anschließend zum N-Hydroxylanilin der Formel Va reduziert und Va mit einer Carbonylverbindung der Formel VI in I umwandelt.

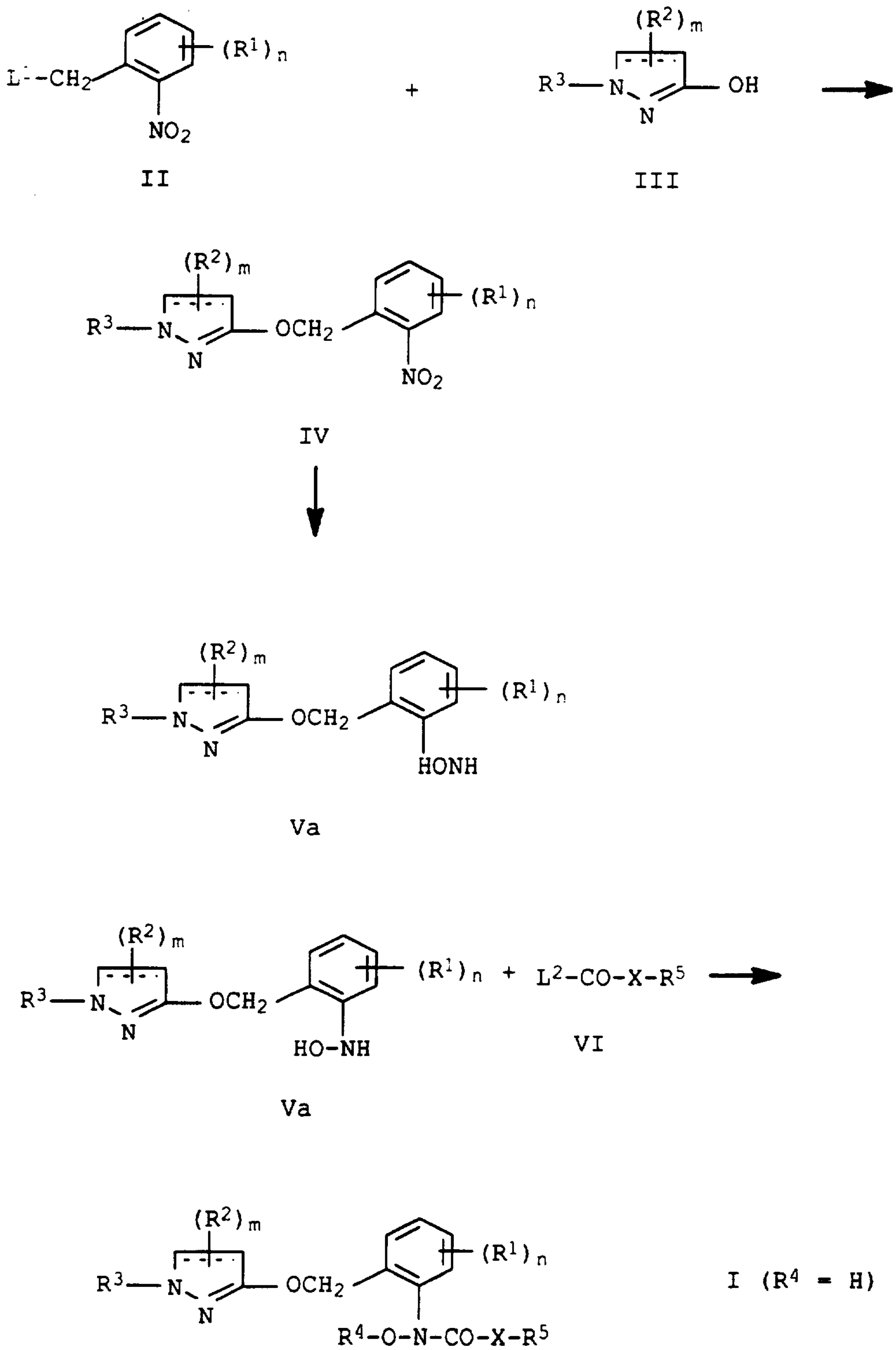

II

III

IV

Va

Va

VI

I ($R^4$ = H)

$L^1$ in der Formel II und $L^2$ in der Formel VI bedeuten jeweils eine nucleophil austauschbare Gruppe, beispielsweise

Halogen (z.B. Chlor, Brom und Iod), oder ein Alkyl oder Arylsulfonat (z.B. Methylsulfonat, Trifluormethylsulfonat, Phenylsulfonat und 4-Methylphenylsulfonat).

Die Veretherung der Verbindungen II und III wird üblicherweise bei Temperaturen von 0°C bis 80°C, vorzugsweise 20°C bis 60°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton, Toluol, Methyl-tert.-butylether und Dimethylformamid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat) sowie Phosphoniumhalogenide (z.B. Tetrabutylphosphoniumchlorid und Tetraphenylphosphoniumbromid) in Betracht.

Es kann für die Umsetzung vorteilhaft sein, zunächst das 3-Hydroxy(dihydro)pyrazol mit der Base in das entsprechende Hydroxylat umzusetzen, welches dann mit dem Benzylderivat umgesetzt wird.

Die für die Herstellung der Verbindungen I benötigten Ausgangsstoffe II sind aus EP-A 513 580 bekannt oder können nach den dort beschriebenen Methoden hergestellt werden [Synthesis 1991, 181; Anal. Chim. Acta 185, 295 (1986); EP-A 336 567].

3-Hydroxypyrazole IIIa und 3-Hydroxydihydropyrazole IIIb sind ebenfalls aus der Literatur bekannt oder können nach den dort beschriebenen Methoden hergestellt werden [IIIa: J. Heterocycl. Chem. 30, 49 (1993), Chem. Ber. 107, 1318 (1974), Chem. Pharm. Bull. 19, 1389 (1971), Tetrahedron Lett. 11, 875 (1970)Chem. Herterocycl. Comp. 5, 527 (1969), Chem. Ber. 102, 3260 (1969), Chem. Ber. 109, 261 (1976), J. Org. Chem. 31, 1538 (1966), Tetrahedron 43, 607 (1987); IIIb: J. Med. Chem. 19, 715 (1976)].

Besonders vorteilhaft erhält man die 3-Hydroxypyrazole IIIa nach dem in der früheren Anmeldung DE Anm. Nr. 4 15 484.4 beschriebenen Verfahren.

Die Reduktion der Nitroverbindungen IV zu den entsprechenden N-Hydroxyanilinen Va erfolgt analog zu literaturbekannten Methoden beispielsweise mit Metallen wie Zink [vgl. Ann. Chem. 316, 278 (1901)] oder mit Wasserstoff (vgl. EP-A 085 890).

Die Umsetzung der N-Hydroxyaniline Va mit den Carbonylverbindungen VI erfolgt unter alkalischen Bedingungen gemäß den vorstehend für die Umsetzung der Verbindungen II mit den 3-Hydroxy(dihydro)pyrazolen III beschriebenen Bedingungen. Insbesondere wird die Umsetzung bei Temperaturen von -10°C bis 30°C durchgeführt. Die bevorzugten Lösungsmittel sind Methylenchlorid, Toluol, tert.-Butylmethylether oder Essigsäureethylester. Die bevorzugten Basen sind Natriumhydrogencarbonat, Kaliumcarbonat oder wäßrige Natriumhydroxid Lösung.

Außerdem erhält man die Verbindungen der Formel I, in denen X für eine direkte Bindung oder Sauerstoff steht, beispielsweise dadurch, daß man ein Tolylderivat der Formel IIa zunächst zum entsprechenden Hydroxyanilin der Formel Vb reduziert, Vb mit einer Carbonylverbindung der Formel VI in das entsprechende Anilid der Formel VII überführt, VII anschließend mit einer Verbindung VIII in das Amid der Formel IX überführt, IX anschließend in das entsprechende Benzylhalogenid X überführt und X in Gegenwart einer Base mit einem 3-Hydroxy(dihydro)pyrazol III in I umwandelt.

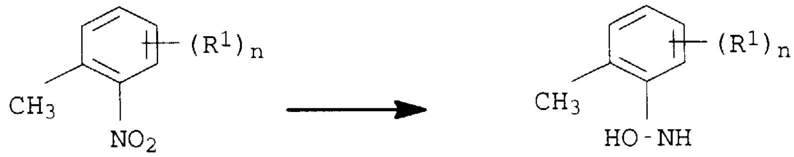

IIa Vb

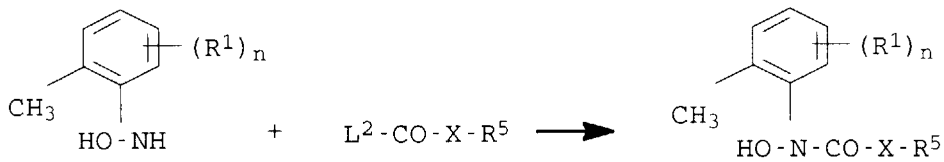

Vb VI VII

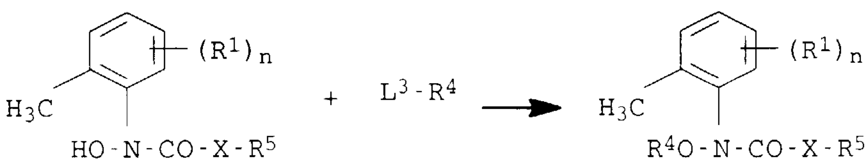

VII VIII IX

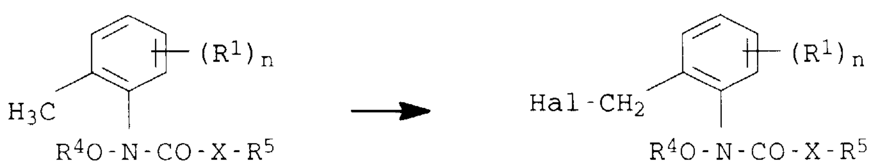

IX X

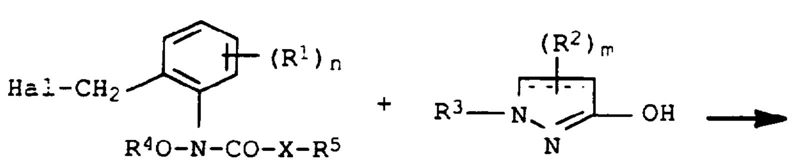

I ($R^4 \neq H$)

X III

In der Formel X bedeutet Hal ein Halogenatom , insbesondere Chlor oder Brom.

$L^3$ in der Formel VIII bedeutet eine nucleophil austauschbare Gruppe, beispielsweise Halogen (z.B. Chlor, Brom und Iod), oder ein Alkyl- oder Arylsulfonat (z.B. Methylsulfonat, Trifluormethylsulfonat, Phenylsulfonat und 4-Methylphenylsulfonat) und $R^4$ steht nicht für Wasserstoff.

Die Umsetzungen erfolgen analog den vorstehend ausgeführten Verfahren.

Die Halogenierung der Verbindungen IX erfolgt radikalisch, wobei als Halogenierungsmittel beispielsweise N-Chlor- oder N-Bromsuccinimid, elementare Halogene (z.B. Chlor oder Brom) oder Thionylchlorid, Sulfurylchlorid, Phosphortri- oder Phosphorpentachlorid und ähnliche Verbindungen eingesetzt werden können. Üblicherweise verwendet

man zusätzlich einen Radikalstarter (z.B. Azobisisobutyronitril) oder man führt die Umsetzung unter Bestrahlung (mit UV-licht) durch. Die Halogenierung erfolgt in an sich bekannter Weise in einem üblichen organischen Verdünnungsmittel.

Die Verbindungen I, in denen $R^4$ nicht Wasserstoff bedeutet, erhält man außerdem dadurch, daß man eine entsprechende Verbindung der Formel I, in der R4 Wasserstof bedeutet, mit einer Verbindung der Formel VIII umsetzt.

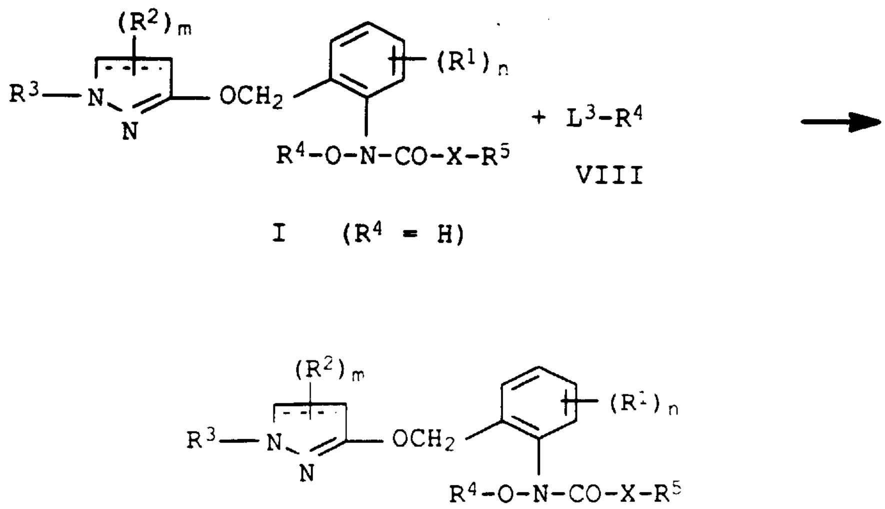

VIII

I ($R^4$ = H)

I ($R^4 \neq$ H)

Die Umsetzung erfolgt in an sich bekannter Weise in einem inerten organischen Lösungsmittel in Gegenwart einer Base bei Temperaturen von 0°C bis 50°C.

Als Basen dienen insbesondere Natriumhydrogencarbonat, Kaliumcarbonat, Natriumhydroxid und wäßrige Natriumhydroxid Lösungen.

Als Lösungsmittel finden insbesondere Aceton, Dimethylformamid, Toluol, tert.-Butylmethylether, Essigsäureethylester und Methanol Verwendung.

Die Verbindungen der Formel I, in denen X für $NR^a$ steht, erhält man vorteilhaft dadurch, daß man ein Benzylanilid der Formel IXa in das entsprechende Benzylhalogenid der Formel Xa überführt, Xa in Gegenwart einer Base mit einem 3-Hydroxy(dihydro)pyrazol der Formel III in eine Verbindung der Formel I.A überführt und I.A anschließend mit einem primären oder sekundären Amin der Formel XI zu I umsetzt.

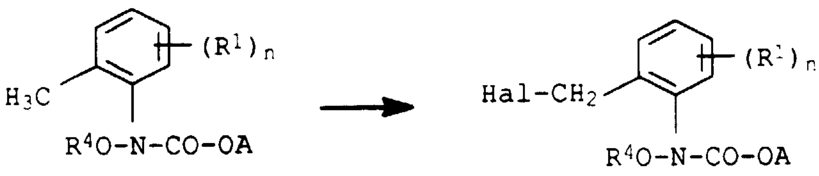

IXa Xa

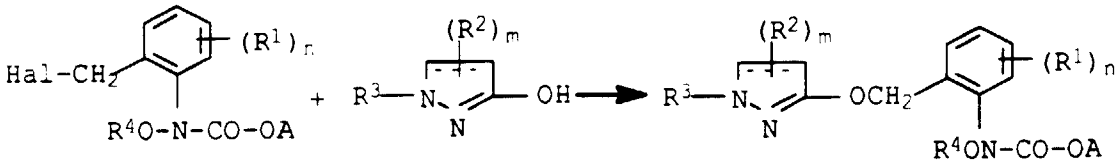

Xa III I.A

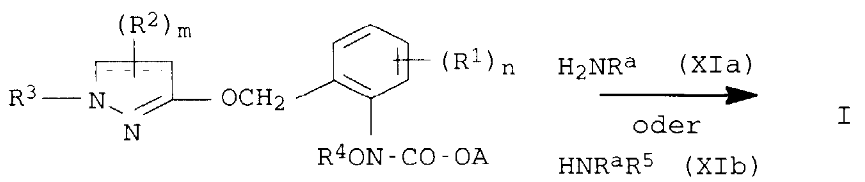


I.A

A in der Formel IXa, Xa, Ia steht für Alkyl (insbesondere $C_1$-$C_6$ -Alkyl) oder Phenyl; Hal in der Formel Xa steht für Halogen (insbesondere Chlor und Brom).

Die Umsetzungen von IXa nach Xa und von Xa nach I.A erfolgen im allgemeinen und im besonderen unter den vorstehend beschriebenen Bedingeungen.

Die Umsetzung der Verbindungen I.A mit den primären oder sekundären Aminen der Formel XIa bzw. XIb erfolgt bei Temperaturen von 0°C bis 100°C in einem inerten Lösungsmittel oder in einem Lösungsmittelgemisch.

Als Lösungsmittel eignen sich insbesondere Wasser, tert.-Butylmethylether und Toluol oder deren Gemische. Es kann vorteilhaft sein, zur Verbesserung der Löslichkeit der Edukte zusätzlich eines der folgenden Lösungsmittel (als Lösungsvermittler) zuzusetzen: Tetrahydrofuran, Methanol, Dimethylformamid und Ethylenglycolether.

Die Amine XIa bzw. XIb werden üblicherweise in einem Überschuß bis zu 100% bezogen auf die Verbindungen X eingesetzt oder können als Lösungsmittel verwendet werden. Es kann im Hinblick auf die Ausbeute vorteilhaft sein, die Umsetzung unter Druck durchzuführen.

Die Herstellung der Verbindungen I erfolgt über Zwischenprodukte der Formel XII

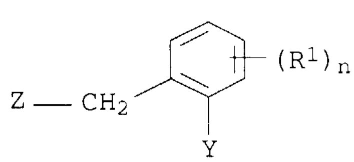

XII

in der die Substituenten und der Index die folgende Bedeutung haben:

n 0, 1, 2, 3 oder 4, wobei die Substituenten $R^1$ verschieden sein können, wenn n größer als 1 ist;

$R^1$ Nitro, Cyano, Halogen,

ggf. subst. Alkyl, Alkenyl, Alkinyl, Alkoxy, Alkenyloxy, Alkinyloxy oder

für den Fall, daß n für 2 steht zusätzlich eine an zwei benachbarte Ringatome gebundene ggf. subst. Brücke, welche drei bis vier Glieder aus der Gruppe 3 oder 4 Kohlenstoffatome, 1 bis 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann;

Y $NO_2$, NHOH- oder $NHOR^4$

$R^4$ ggf. subst. Alkyl, Alkenyl, Alkinyl, Cycloalkyl, Cycloalkenyl, Alkylcarbonyl oder Alkoxycarbonyl;

Z Wasserstoff, Hydroxy, Mercapto, Cyano, Nitro, Halogen, $C_1$-$C_6$-Alkylsulfonyl, ggf. subst. Arylsulfonyl oder eine Gruppe $Z^a$

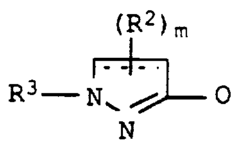

$Z^a$

m 0, 1 oder 2, wobei die Substituenten $R^2$ verschieden sein können, wenn m größer als 1 ist;

$R^2$ Nitro, Cyano, Halogen, $C_1$-$C_4$-Alkyl, $C_1$-$C_4$-Halogenalkyl, $C_1$-$C_4$-Alkoxy, $C_1$-$C_4$-Alkylthio oder $C_1$-$C_4$-Alkoxycarbonyl;

$R^3$ ggf. subst. Alkyl, Alkenyl oder Alkinyl;

ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, oder

ein ggf. subst. ein- oder zweikerniger aromatischer Rest, welcher neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

Insbesondere sind bei der Herstellung Zwischenprodukte der Formel XII bevorzugt, in denen Y für NHOH und Z für die Gruppe $Z^a$ steht.

Außerdem sind bei der Herstellung Zwischenprodukte der Formel IX bevorzugt, in denen Y für NO2 und Z für die Gruppe $Z^a$ steht.

Im Hinblick auf die Herstellung der Verbindungen I, in denen X für $NR^a$ steht werden Zwischenprodukte der allgemeinen Formel XIII

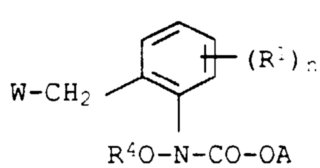

XIII

bevorzugt, wobei die Substituenten $R^1$ und $R^4$ sowie der Index n die eingangs gegebene Bedeutung haben und die Substituenten W und A die folgende Bedeutung haben:

W Wasserstoff, Halogen oder $Z^a$, und

A Alkyl oder Phenyl.

Insbesondere sind hierbei Verbindungen XIII bevorzugt, bei denen der Substituenten W für Wasserstoff, Chlor, Brom oder $Z^a$ steht.

Außerdem sind solche Verbindungen XIII bevorzugt, bei denen der Substituent A für $C_1$-$C_6$-Alkyl steht.

Insbesondere sind auch solche Verbindungen XIII besonders bevorzugt, in denen der Substituent A für Phenyl steht.

Gleichermaßen bevorzugt sind solche Verbindungen XIII, in denen $R^4$ für Wasserstoff, Methyl oder Ethyl steht.

Daneben werden Verbindungen XIII bevorzugt, in denen n für 0 oder 1 steht.

Besonders bevorzugt sind solche Verbindungen XIII, in denen die Substituenten und der Index die folgende Bedeutung haben:

n 0,
W Wasserstoff, Chlor, Brom oder $Z^a$,
$R^4$ Wasserstoff, Methyl oder Ethyl und
A Phenyl.

Die Verbindungen I können saure oder basische Zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schwefelsäure, Salpetersäure), organische Säuren (z.B. Ameisensäure, Essigsäure, Oxalsäure, Malonsäure, Milchsäure, Äpfelsäure, Bernsteinsäure, Weinsäure, Zitronensäure, Salizylsäure, p-Toluolsulfonsäure, Dodecylbenzolsulfonsäure) oder andere protonenacide Verbindugnen (z.B. Saccharin).
Basen für Basenadditionsprodukte sind u.a. Oxide, Hydroxide, Carbonate oder Hydrogencarbonate von Alkalimetallen oder Erdalkaimetallen (z.B. Kalium- oder Natriumhydroxyd oder -carbonat) oder Ammoniumverbindungen (z.B. Ammoniumhydroxyd).

Bei den in den vorstehenden Formeln angegebenen Definitionen der Symbole wurden z.T. Sammelbegriffe verwendet, die allgemein repräsentativ für die folgenden Substituenten stehen:

Halogen: Fluor, Chlor, Brom und Jod;

Alkyl: gesättigte, geradkettige oder verzweigte Kohlenwasserstoffreste mit 1 bis 4 oder 10 Kohlenstoffatomen, z. B. Methyl, Ethyl, Propyl, 1-Methylethyl, Butyl, 1-Methyl-propyl, 2-Methylpropyl und 1,1-Dimethylethyl;

Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

Alkylcarbonyl: geradkettige oder verzweigte Alkylgruppen, insbesondere mit 1 bis 10 Kohlenstoffatomen (wie vorstehend genannt), welche über eine Carbonylgruppe (-CO-) an das Gerüst gebunden sind;

Alkoxy: geradkettige oder verzweigte Alkylgruppen mit 1 bis 4 oder 10 Kohlenstoffatomen (wie vorstehend genannt), welche über ein Sauerstoffatom (-O-) an das Gerüst gebunden sind;

Alkoxycarbonyl: geradkettige oder verzweigte Alkoxygruppen mit 1 bis 4 Kohlenstoffatomen (wie vorstehend genannt), welche über eine Carbonylgruppe (-CO-) an das Gerüst gebunden sind;

Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;

ggf. subst. Alkyl: gesättigte, geradkettige oder verzweigte Kohlenwasserstoffreste, insbesondere mit 1 bis 10 Kohlenstoffatomen, z.B. $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl,

1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

ggf. subst. Alkenyl: ungesättigte, geradkettige oder verzweigte Kohlenwasserstoffreste, insbesondere mit 2 bis 10 Kohlenstoffatomen und einer Doppelbindung in einer beliebigen Position, z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;

ggf. subst. Alkenyloxy: geradkettige oder verzweigte Alkenylgruppen mit 3 bis 10 Kohlenstoffatomen (wie vorstehend genannt), welche über ein Sauerstoffatom (-O-) an das Gerüst gebunden sind;

Alkinyl: geradkettige oder verzweigte Kohlenwasserstoffgruppen, insbesondere mit 2 bis 20 Kohlenstoffatomen und einer Dreifachbindung in einer beliebigen Position, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

ggf. subst. Alkinyloxy: geradkettige oder verzweigte Alkinylgruppen mit 3 bis 10 Kohlenstoffatomen (wie vorstehend genannt), welche über ein Sauerstoffatom (-O-) an das Gerüst gebunden sind;

ggf. subst. Cycloalkyl: mono- oder bicyclische Kohlenwasserstoffreste mit 3 bis 10 Kohlenstoffatomen, z.B. $C_3$-$C_{10}$-(Bi)cycloalkyl wie Cyclopropyl, Cyclobutyl, Cyclopentyl, Cyclohexyl, Cycloheptyl, Bornanyl, Norbornanyl, Dicyclohexyl, Bicyclo[3,3,0]octyl, Bicyclo[3,2,1]octyl, Bicyclo[2,2,2]octyl oder Bicyclo[3,3,1]nonyl;

ggf. subst. Cycloalkenyl: mono- oder bicyclische Kohlenwasserstoffreste mit 5 bis 10 Kohlenstoffatomen und einer Doppelbindung in einer beliebigen Ringposition, z.B. $C_5$-$C_{10}$-(Bi)cycloalkenyl wie Cyclopentenyl, Cyclohexenyl, Cycloheptenyl, Bornenyl, Norbornenyl, Dicyclohexenyl und Bicyclo[3,3,0]octenyl;

eine an zwei benachbarte Ringatome gebundene ggf. subst. Brücke, welche drei bis vier Glieder aus der Gruppe 3 oder 4 Kohenstoffatome, 1 bis 3 Kohlenstoffatome und 1 oder 2 Stickstoff-, Sauerstoff- und/oder Schwefelatome, wobei diese Brücke gemeinsam mit dem Ring an den sie gebunden ist einen partiell ungesättigten oder aromatischen Rest bilden kann: Brücken, die mit dem Ring, an den sie gebunden sind beispielsweise eines der folgenden Systeme bilden: Chinolinyl, Benzofuranyl und Naphthyl;

ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;

oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;

Sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz "ggf. subst" in Bezug auf Alkyl-, Alkenyl- und Alkinylgruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein) und/oder einen bis drei, insbesondere einen, der folgenden Reste tragen können:

$C_1$-$C_6$-Alkoxy, $C_1$-$C_6$-Halogenalkoxy, $C_1$-$C_6$-Alkylthio, $C_1$-$C_6$Halogenalkylthio, $C_1$-$C_6$-Alkylamino, Di-$C_1$-$C_6$-alkylamino, C2-$C_6$-Alkenyloxy, $C_2$-$C_6$-Halogenalkenyloxy, $C_2$-$C_6$-Alkinyloxy, $C_2$-$C_6$-Halogenalkinyloxy, $C_3$-$C_6$-Cycloalkyl, $C_3$-$C_6$-Cycloalkyloxy, $C_3$-$C_6$-Cycloalkenyl, C3-C6-Cycloalkenyloxy,

oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann (wie vorstehend genannt), welches direkt oder über ein Sauerstoffatom (-O-), ein Schwefelatom (-S-) oder eine Aminogruppe (-$NR^a$-) an den Substituenten gebunden sein kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;

Sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz "ggf. subst" in Bezug auf die cyclischen (gesättigten, ungesättigtern oder aromatischen) Gruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein) und/oder einen bis drei Reste tragen können.

Die bei den Resten genannten ein- oder zweikernigen aromatischen oder heteroaromatischen Systeme können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweikernigen aromatischen oder heteroaromatischen Systeme können neben den bezeichneten Halogenatomen ein bis drei der folgenden Substituenten tragen:

Nitro;

Cyano, Thiocyanato;

Alkyl, besonders $C_1$-$C_6$-Alkyl wie vorstehend genannt, vorzugsweise Methyl, Ethyl, 1-Methylethyl, 1,1-Dimethylethyl, Butyl, Hexyl, insbesondere Methyl und 1-Methylethyl;

$C_1$-$C_4$-Halogenalkyl, wie vorstehend genannt, vorzugsweise Trichlormethyl, Difluormethyl, Trifluormethyl, 2,2-Difluorethyl, 2,2,2-Trifluorethyl und Pentafluorethyl;

$C_1$-$C_4$-Alkoxy, vorzugsweise Methoxy, Ethoxy, 1-Methylethoxy und 1,1-Dimethylethoxy, insbesondere Methoxy;

$C_1$-$C_4$-Halogenalkoxy, besonders $C_1$-$C_2$-Halogenalkoxy, vorzugsweise Difluormethyloxy, Trifluormethyloxy und 2,2,2-Trifluorethyloxy, insbesondere Difluormethyloxy;

$C_1$-$C_4$-Alkylthio, vorzugsweise Methylthio und 1-Methylethylthio, insbesondere Methylthio;

$C_1$-$C_4$-Alkylamino wie Methylamino, Ethylamino, Propylamino, 1-Methylethylamino, Butylamino, 1-Methylpropylamino, 2-Methylpropylamino und 1,1-Dimethylethylamino, vorzugsweise Methylamino und 1,1-Dimethylethylamino, insbesondere Methylamino,

Di-$C_1$-$C_4$-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl) amino, N-Ethyl-N-methylamino, N-Methyl-N-propylamino, N-Methyl-N-(1-methylethyl)amino, N-Butyl-N-methylamino, N-Methyl-N- (1-methylpropyl) amino, N-Methyl-N- (2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)N-(2-methylpropyl)amino, N-(1,1-Dimethylethyl)-N-(1-methylpropyl)amino und N-(1,1-Dimethylethyl)-N-(2-methylpropyl)amino, vorzugsweise N,N-Dimethylamino und N,N-Diethylamino, insbesondere N,N-Dimethylamino;

$C_1$-$C_6$-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl und

1,1-Dimethylcarbonyl, insbesondere Ethylcarbonyl;

$C_1$-$C_6$-Alkoxycarbonyl wie Methoxycarbonyl, Ethoxycarbonyl, Propyloxycarbonyl, l-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise Methoxycarbonyl, Ethoxycarbonyl und 1,1-Dimethylethoxycarbonyl, insbesondere Ethoxycarbonyl;

$C_1$-$C_6$-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise Methylamincarbonyl und Ethylamincarbonyl, insbesondere Methylaminocarbonyl;

Di-$C_1$-$C_6$-alkylaminocarbonyl, besonders Di-$C_1$-$C_4$-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl) aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N- (1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise N,N-Dimethylaminocarbonyl und N,N-Diethylamincarbonyl, insbesondere N,N-Dimethylaminocarbonyl;

$C_1$-$C_6$-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-Ethyl-2-methylpropylcarboxyl, vorzugsweise Methylcarboxyl, Ethylcarboxyl und 1,1-Dimethylethylcarbonyl, insbesondere Methylcarboxyl und 1,1-Dimethylethylcarboxyl;

$C_1$-$C_6$-Alkylcarbonylamino wie Methylcarbonylamino, Ethylcarbonylamino, Propylcarbonylamino, 1-Methylethylcarbonylamino, Butylcarbonylamino, 1-Methylpropylcarbonylamino, 2-Methylpropylcarbonylamino, 1,1-Dimethylethylcarbonylamino, Pentylcarbonylamino, 1-Methylbutylcarbonylamino, 2-Methylbutylcarbonylamino, 3-Methylbutylcarbonylamino, 2,2-Dimethylpropylcarbonylamino, 1-Ethylpropylcarbonylamino, Hexylcarbonylamino, 1,1-Dimethylpropylcarbonylamino, 1,2-Dimethylpropylcarbonylamino, 1-Methylpentylcarbonylamino, 2-Methylpentylcarbonylamino, 3-Methylpentylcarbonylamino, 4-Methylpentylcarbonylamino, 1,1-Dimethylbutylcarbonylamino, 1,2-Dimethylbutylcarbonylamino, 1,3-Dimethylbutylcarbonylamino, 2,2-Dimethylbutylcarbonylamino, 2,3-Dimethylbutylcarbonylamino, 3,3-Dimethylbutylcarbonylamino, 1-Ethylbutylcarbonylamino, 2-Ethylbutylcarbonylamino, 1,1,2-Trimethylpropylcarbonylamino, 1,2,2-Trimethylpropylcarbonylamino, 1-Ethyl-1-methylpropylcarbonylamino und 1-Ethyl-2-methylpropylcarbonylamino, vorzugsweise Methylcarbonylamino und Ethylcarbonylamino, insbesondere Ethylcarbonylamino;

$C_3$-$C_7$-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;

$C_3$-$C_7$-Cycloalkoxy wie Cyclopropyloxy, Cyclobutyloxy, Cyclopentyloxy, Cyclohexyloxy und Cycloheptyloxy, vorzugsweise Cyclopentyloxy und Cyclohexyloxy, insbesondere Cyclohexyloxy;

$C_3$-$C_7$-Cycloalkylthio wie Cyclopropylthio, Cyclobutylthio, Cyclopentylthio, Cyclohexylthio und Cycloheptylthio, vorzugsweise Cyclohexylthio;

$C_3$-$C_7$-Cycloalkylamino wie Cyclopropylamino, Cyclobutylamino, Cyclopentylamino, Cyclohexylamino und Cycloheptylamino, vorzugsweise Cyclopropylamino und Cyclohexylamino, insbesondere Cyclopropylamino;

Die ein- oder zweikernigen aromatischen oder heteroaromatischen Systeme können neben den vorstehend genannten Substituenten auch einen Rest -CR'=NOR" tragen, wobei die Reste R' und R" für die folgenden Gruppen stehen:

R' Wasserstoff, Cyano, Alkyl (vorzugsweise $C_1$-$C_6$-Alkyl, insbesondere $C_1$-$C_4$-Alkyl), Haloalkyl (vorzugsweise $C_1$-$C_4$-Haloalkyl, insbesondere $C_1$-$C_2$-Haloalkyl), Alkenyl (vorzugsweise $C_2$-$C_6$-Alkenyl, insbesondere $C_2$-$C_4$-Alkenyl), Haloalkenyl (vorzugsweise $C_2$-$C_6$-Haloalkenyl, insbesondere $C_2$-$C_4$-Haloalkenyl), Alkinyl (vorzugsweise $C_2$-$C_6$-Alkinyl, insbesondere $C_2$-$C_4$-Alkinyl), Haloalkinyl (vorzugsweise $C_2$-$C_6$-Haloalkinyl, insbesondere $C_2$-$C_4$-Haloalkinyl) und Cycloalkyl (vorzugsweise $C_3$-$C_8$-Cycloalkyl, insbesondere $C_3$-$C_6$-Cycloalkyl);

R" Alkyl (vorzugsweise $C_1$-$C_6$-Alkyl, insbesondere $C_1$-$C_4$-Alkyl), Haloalkyl (vorzugsweise $C_1$-$C_4$-Haloalkyl, insbesondere $C_1$-$C_2$-Haloalkyl), Alkenyl (vorzugsweise $C_2$-$C_6$-Alkenyl, insbesondere $C_2$-$C_4$-Alkenyl), Haloalkenyl (vorzugsweise $C_2$-$C_6$-Haloalkenyl, insbesondere $C_2$-$C_4$-Haloalkenyl), Alkinyl (vorzugsweise $C_2$-$C_6$-Alkinyl, insbesondere $C_2$-$C_4$-Alkinyl), Haloalkinyl (vorzugsweise $C_2$-$C_6$-Haloalkinyl, insbesondere $C_2$-$C_4$-Haloalkinyl) und Cycloalkyl (vorzugsweise $C_3$-$C_8$-Cycloalkyl, insbesondere $C_3$-$C_6$-Cycloalkyl).

Im Hinblick auf ihre biologische Wirkung sind Verbindungen I bevorzugt, in denen -- für eine Doppelbindung steht.

Desweiteren sind Verbindungen I bevorzugt, in denen in der -- für eine Einfachbindung steht.

Gleichermaßen sind Verbindungen I bevorzugt, in denen n für 0 oder 1, insbesondere für 0, steht.

Außerdem werden Verbindungen I bevorzugt, in denen $R^1$ für Halogen, $C_1$-$C_4$-Alkyl, $C_1$-$C_2$-Halogenalkyl, $C_1$-$C_4$-Alkoxy oder $C_1$-$C_2$-Halogenalkoxy steht.

Daneben werden Verbindungen I bevorzugt, in denen m 0 oder 1 bedeutet.

Gleichermaßen werden Verbindungen I bevorzugt, in denen $R^2$ Nitro, Halogen, $C_1$-$C_4$-Alkyl, $C_1$-$C_4$-Halogenalkyl, $C_1$-$C_4$-Alkoxy oder $C_1$-$C_4$-Alkoxycarbonyl steht.

Desweiteren werden Verbindungen I bevorzugt, in denen $R^3$ für $C_1$-$C_4$-Alkyl oder $C_3$-$C_6$-Cycloalkyl steht.

Außerdem werden Verbindungen I bevorzugt, in denen $R^3$ für einen ggf. subst. ein- oder zweikernigen aromatischen Rest steht, welcher neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

Insbesondere werden Verbindungen I bevorzugt, in denen $R^3$ für Phenyl oder Benzyl steht, wobei der Phenylrest partiell oder vollständig halogeniert sein kann und/oder

- ein bis drei der folgenden Reste: Cyano, Nitro, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxy-$C_1$-$C_4$-alkyl, $C_3$-$C_6$-Cycloalkyl, $C_1$-$C_4$-Alkylcarbonyl, $C_1$-$C_4$-Alkoxycarbonyl, Phenyl, Phenoxy und Phenyl-$C_1$-$C_4$-alkoxy, wobei die Phenylringe ihrerseits partiell oder vollständig halogeniert sein können und/oder ein bis drei der folgenden Reste tragen können: Cyano, Nitro, $C_1$-$C_4$-Alkyl, $C_1$-$C_2$-Haloqenalkyl, $C_1$-$C_4$-Alkoxy, $C_1$-$C_2$-Halogenalkoxy, $C_3$-$C_6$-Cycloalkyl, $C_1$-$C_4$-Alkylcarbonyl oder $C_1$-$C_4$-Alkoxycarbonyl, und/oder
- eine Gruppe CR'=NOR", in der R' Wasserstoff oder $C_1$-$C_4$-Alkyl bedeutet und R" für $C_1$-$C_6$-Alkyl steht, tragen kann und/oder
- zwei benachbarte C-Atome des Phenylrings über eine Oxy-$C_1$-$C_3$-alkoxy-Brücke oder eine Oxy-$C_1$-$C_3$-halogenalkoxy-Brücke verbrückt sind.

Außerdem werden Verbindungen I insbesondere bevorzugt, in denen $R^3$ für Pyridyl oder Pyrimidyl steht, wobei der Pyridylring partiell oder vollständig halogeniert sein kann und/oder ein bis drei der folgenden Reste tragen kann: Cyano, Nitro, $C_1$-$C_4$-Alkyl, $C_1$-$C_2$-Haloqenalkyl, $C_1$-$C_4$-Alkoxy, $C_1$-$C_2$-Halogenalkoxy, $C_3$-$C_6$-Cycloalkyl, $C_1$-$C_4$-Alkylcarbonyl oder $C_1$-$C_4$-Alkoxycarbonyl.

Daneben werden Verbindungen I bevorzugt, in denen $R^4$ für Wasserstoff, $C_1$-$C_4$-Alkyl oder $C_1$-$C_2$-Halogenalkyl steht.

Außerdem werden Verbindungen I bevorzugt, in denen $R^5X$ für Methyl, Ethyl, Methoxy oder Methylamino steht.

Beispiele für insbesondere bevorzugte Verbindungen I sind in den Tabellen zusammengestellt.

Tabelle 1

Verbindungen der allgemeinen Formel I.1, in denen $R^4$ für Methyl steht, $R^5X$ Methyl bedeutet und $R^x_p$ für eine Verbindung einer Zeile der Tabelle A entspricht

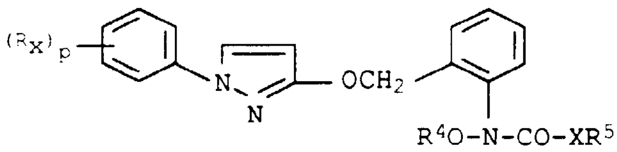

I.1

Tabelle 2

Verbindungen der allgemeinen Formel I.1, in denen $R^4$ für Methyl steht, $R^5X$ Ethyl bedeutet und $R^x_p$ für eine Verbindung einer Zeile der Tabelle A entspricht

Tabelle 3

Verbindungen der allgemeinen Formel I.2, in denen $R^4$ für Methyl steht, $R^5X$ Methyl bedeutet und $R^x_p$ für eine Verbindung einer Zeile der Tabelle A entspricht

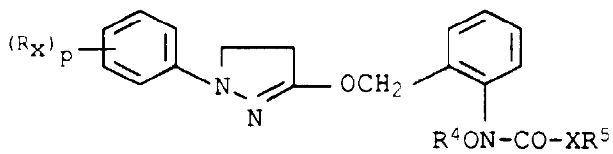

I.2

Tabelle 4

Verbindungen der allgemeinen Formel I.2, in denen $R^4$ für Methyl steht, $R^5X$ Ethyl bedeutet und $R^x_p$ für eine Verbindung einer Zeile der Tabelle A entspricht

Tabelle 5

Verbindungen der allgemeinen Formel I.1, in denen $R^4$ für Methyl steht, $R^5X$ Methoxy bedeutet und $R^x_p$ für eine Verbindung einer Zeile der Tabelle A entspricht

Tabelle 6

Verbindungen der allgemeinen Formel I.2, in denen $R^4$ für Methyl steht, $R^5X$ Methoxy bedeutet und $R^x_p$ für eine Verbindung einer Zeile der Tabelle A entspricht

Tabelle 7

Verbindungen der allgemeinen Formel I.1, in denen $R^4$ für Methyl steht, $R^5X$ Methylamino bedeutet und $R^x{}_p$ für eine Verbindung einer Zeile der Tabelle A entspricht

Tabelle 8

Verbindungen der allgemeinen Formel I.2, in denen $R^4$ für Methyl steht, $R^5X$ Methylamino bedeutet und $R^x{}_p$ für eine Verbindung einer Zeile der Tabelle A entspricht

Tabelle 9

Verbindungen der allgemeinen Formel I.3, in denen $R^4$ für Methyl steht, $R^5X$ Methyl bedeutet, $R^y$ Wasserstoff bedeutet, $R^z$ Chlor bedeutet und $R^x{}_p$ für eine Verbindung einer Zeile der Tabelle A entspricht

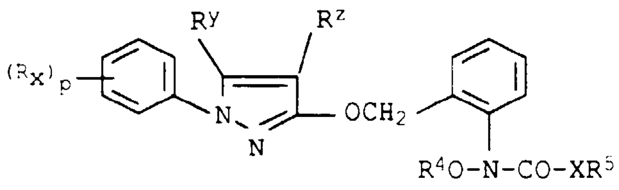

I.3

Tabelle 10

Verbindungen der allgemeinen Formel I.3, in denen $R^4$ für Methyl steht, $R^5X$ Ethyl bedeutet, $R^y$ Wasserstoff bedeutet, $R^z$ Chlor bedeutet und $R^x{}_p$ für eine Verbindung einer Zeile der Tabelle A entspricht

Tabelle 11

Verbindungen der allgemeinen Formel I.3, in denen $R^4$ für Methyl steht, $R^5X$ Methoxy bedeutet, $R^y$ Wasserstoff bedeutet, $R^z$ Chlor bedeutet und $R^x{}_p$ für eine Verbindung einer Zeile der Tabelle A entspricht

Tabelle 12

Verbindungen der allgemeinen Formel I.3, in denen $R^4$ für Methyl steht, $R^5X$ Methylamino bedeutet, $R^y$ Wasserstoff bedeutet, $R^z$ Chlor bedeutet und $R^x{}_p$ für eine Verbindung einer Zeile der Tabelle A entspricht

Tabelle 13

Verbindungen der allgemeinen Formel I.3, in denen $R^4$ für Methyl steht, $R^5X$ Methyl bedeutet, $R^y$ Methyl bedeutet, $R^z$ Wasserstoff bedeutet und $R^x{}_p$ für eine Verbindung einer Zeile der Tabelle A entspricht

Tabelle 14

Verbindungen der allgemeinen Formel I.3, in denen $R^4$ für Methyl steht, $R^5X$ Ethyl bedeutet, $R^y$ Methyl bedeutet, $R^z$ Wasserstoff bedeutet und $R^x{}_p$ für eine Verbindung einer Zeile der Tabelle A entspricht

Tabelle 15

Verbindungen der allgemeinen Formel I.3, in denen $R^4$ für Methyl steht, $R^5X$ Methoxy bedeutet, $R^y$ Methyl bedeutet, $R^z$ Wasserstoff bedeutet und $R^x{}_p$ für eine Verbindung einer Zeile der Tabelle A entspricht

Tabelle 16

Verbindungen der allgemeinen Formel I.3, in denen $R^4$ für Methyl steht, $R^5X$ Methylamino bedeutet, $R^y$ Methyl bedeutet, $R^z$ Wasserstoff bedeutet und $R^x{}_p$ für eine Verbindung einer Zeile der Tabelle A entspricht

Tabelle 17

Verbindungen der allgemeinen Formel I.3, in denen $R^4$ für Methyl steht, $R^5X$ Methyl bedeutet, $R^y$ Trifluormethyl bedeutet, $R^z$ Wasserstoff bedeutet und $R^x_p$ für eine Verbindung einer Zeile der Tabelle A entspricht

Tabelle 18

Verbindungen der allgemeinen Formel I.3, in denen $R^4$ für Methyl steht, $R^5X$ Ethyl bedeutet, $R^y$ Trifluormethyl bedeutet, $R^z$ Wasserstoff bedeutet und $R^x_p$ für eine Verbindung einer Zeile der Tabelle A entspricht

Tabelle 19

Verbindungen der allgemeinen Formel I.3, in denen $R^4$ für Methyl steht, $R^5X$ Methoxy bedeutet, $R^y$ Trifluormethyl bedeutet, $R^z$ Wasserstoff bedeutet und $R^x_p$ für eine Verbindung einer Zeile der Tabelle A entspricht

Tabelle 20

Verbindungen der allgemeinen Formel I.3, in denen $R^4$ für Methyl steht, $R^5X$ Methylamino bedeutet, $R^y$ Trifluormethyl bedeutet, $R^z$ Wasserstoff bedeutet und $R^x_p$ für eine Verbindung einer Zeile der Tabelle A entspricht

Tabelle 21

Verbindungen der allgemeinen Formel I.4, in denen $R^5X$ Methyl bedeutet und die Kombination der Substituenten $R^1$, $R^y$, $R^2$, $R^3$ und $R^4$ für eine Verbindung jeweils einer Zeile der Tabelle B entspricht

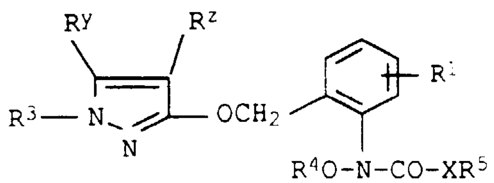

I.4

Tabelle 22

Verbindungen der allgemeinen Formel I.4, in denen $R^5X$ Ethyl bedeutet und die Kombination der Substituenten $R^1$, $R^y$, $R^2$, $R^3$ und $R^4$ für eine Verbindung jeweils einer Zeile der Tabelle B entspricht

Tabelle 23

Verbindungen der allgemeinen Formel I.4, in denen $R^5X$ Methoxy bedeutet und die Kombination der Substituenten $R^1$, $R^y$, $R^2$, $R^3$ und $R^4$ für eine Verbindung jeweils einer Zeile der Tabelle B entspricht

Tabelle 24

Verbindungen der allgemeinen Formel I.4, in denen $R^5X$ Methylamino und bedeutet und die Kombination der Substituenten $R^1$, $R^y$, $R^2$, $R^3$ und $R^4$ für eine Verbindung jeweils einer Zeile der Tabelle B entspricht

Tabelle 25

Verbindungen der allgemeinen Formel I.1, in denen $R^4$ für Wasserstoff steht, $R^5X$ Methyl bedeutet und $R^x_p$ für eine Verbindung einer Zeile der Tabelle A entspricht

Tabelle 26

Verbindungen der allgemeinen Formel I.1, in denen $R^4$ für Wasserstoff steht, $R^5X$ Ethyl bedeutet und $R^x_p$ für eine Verbindung einer Zeile der Tabelle A entspricht

Tabelle 27

Verbindungen der allgemeinen Formel I.2, in denen $R^4$ für Wasserstoff steht, $R^5X$ Methyl bedeutet und $R^x_p$ für eine Verbindung einer Zeile der Tabelle A entspricht

Tabelle 28

Verbindungen der allgemeinen Formel I.2, in denen $R^4$ für Wasserstoff steht, $R^5X$ Ethyl bedeutet und $R^x_p$ für eine Verbindung einer Zeile der Tabelle A entspricht

Tabelle 29

Verbindungen der allgemeinen Formel I.1, in denen $R^4$ für Wasserstoff steht, $R^5X$ Methoxy bedeutet und $R^x_p$ für eine Verbindung einer Zeile der Tabelle A entspricht

Tabelle 30

Verbindungen der allgemeinen Formel I.2, in denen $R^4$ für Wasserstoff steht, $R^5X$ Methoxy bedeutet und $R^x_p$ für eine Verbindung einer Zeile der Tabelle A entspricht

Tabelle 31

Verbindungen der allgemeinen Formel I.1, in denen $R^4$ für Wasserstoff steht, $R^5X$ Methylamino bedeutet und $R^x_p$ für eine Verbindung einer Zeile der Tabelle A entspricht

Tabelle 32

Verbindungen der allgemeinen Formel I.2, in denen $R^4$ für Wasserstoff steht, $R^5X$ Methylamino bedeutet und $R^x_p$ für eine Verbindung einer Zeile der Tabelle A entspricht

Tabelle 33

Verbindungen der allgemeinen Formel I.3, in denen $R^4$ für Wasserstoff steht, $R^5X$ Methyl bedeutet, $R^y$ Wasserstoff bedeutet, $R^z$ Chlor bedeutet und $R^x_p$ für eine Verbindung einer Zeile der Tabelle A entspricht

Tabelle 34

Verbindungen der allgemeinen Formel I.3, in denen R4 für Wasserstoff steht, $R^5X$ Ethyl bedeutet, $R^y$ Wasserstoff bedeutet, $R^z$ Chlor bedeutet und $R^x_p$ für eine Verbindung einer Zeile der Tabelle A entspricht

Tabelle 35

Verbindungen der allgemeinen Formel I.3, in denen R4 für Wasserstoff steht, $R^5X$ Methoxy bedeutet, $R^y$ Wasserstoff bedeutet, $R^z$ Chlor bedeutet und $R^x_p$ für eine Verbindung einer Zeile der Tabelle A entspricht

Tabelle 36

Verbindungen der allgemeinen Formel I.3, in denen $R^4$ für Wasserstoff steht, $R^5X$ Methylamino bedeutet, $R^y$ Wasserstoff bedeutet, $R^z$ Chlor bedeutet und $R^x_p$ für eine Verbindung einer Zeile der Tabelle A entspricht

Tabelle 37

Verbindungen der allgemeinen Formel I.3, in denen R4 für Wasserstoff steht, $R^5X$ Methyl bedeutet, $R^y$ Methyl bedeutet, $R^z$ Wasserstoff bedeutet und $R^x_p$ für eine Verbindung einer Zeile der Tabelle A entspricht

Tabelle 38

Verbindungen der allgemeinen Formel I.3, in denen $R^4$ für Wasserstoff steht, $R^5X$ Ethyl bedeutet, $R^y$ Methyl bedeutet, $R^z$ Wasserstoff bedeutet und $R^x{}_p$ für eine Verbindung einer Zeile der Tabelle A entspricht

Tabelle 39

Verbindungen der allgemeinen Formel I.3, in denen $R^4$ für Wasserstoff steht, $R^5X$ Methoxy bedeutet, $R^y$ Methyl bedeutet, $R^z$ Wasserstoff bedeutet und $R^x{}_p$ für eine Verbindung einer Zeile der Tabelle A entspricht

Tabelle 40

Verbindungen der allgemeinen Formel I.3, in denen $R^4$ für Wasserstoff steht, $R^5X$ Methylamino bedeutet, $R^y$ Methyl bedeutet, $R^z$ Wasserstoff bedeutet und $R^x{}_p$ für eine Verbindung einer Zeile der Tabelle A entspricht

Tabelle 41

Verbindungen der allgemeinen Formel I.3, in denen $R^4$ für Wasserstoff steht, $R^5X$ Methyl bedeutet, $R^y$ Trifluormethyl bedeutet, $R^z$ Wasserstoff bedeutet und $R^x{}_p$ für eine Verbindung einer Zeile der Tabelle A entspricht

Tabelle 42

Verbindungen der allgemeinen Formel I.3, in denen $R^4$ für Wasserstoff steht, $R^5X$ Ethyl bedeutet, $R^y$ Trifluormethyl bedeutet, $R^z$ Wasserstoff bedeutet und $R^x{}_p$ für eine Verbindung einer Zeile der Tabelle A entspricht

Tabelle 43

Verbindungen der allgemeinen Formel I.3, in denen $R^4$ für Wasserstoff steht, $R^5X$ Methoxy bedeutet, $R^y$ Trifluormethyl bedeutet, $R^z$ Wasserstoff bedeutet und $R^x{}_p$ für eine Verbindung einer Zeile der Tabelle A entspricht

Tabelle 44

Verbindungen der allgemeinen Formel I.3, in denen $R^4$ für Wasserstoff steht, $R^5X$ Methylamino bedeutet, $R^y$ Trifluormethyl bedeutet, $R^z$ Wasserstoff bedeutet und $R^x{}_p$ für eine Verbindung einer Zeile der Tabelle A entspricht

Tabelle A

| Nummer | $R^x{}_p$ |
|---|---|
| 1 | H |
| 2 | 2-F |
| 3 | 3-F |
| 4 | 4-F |
| 5 | 2,4-$F_2$ |
| 6 | 2,4,6-$F_3$ |
| 7 | 2,3,4,5,6-$F_5$ |
| 8 | 2,3-$F_2$ |
| 9 | 2-Cl |
| 10 | 3-Cl |
| 11 | 4-Cl |
| 12 | 2,3-$Cl_2$ |
| 13 | 2,4-$Cl_2$ |
| 14 | 2,5-$Cl_2$ |
| 15 | 2,6-$Cl_2$ |
| 16 | 3,4-$Cl_2$ |

Tabelle A (fortgesetzt)

| Nummer | $R^x_p$ |
|---|---|
| 17 | 3,5-$Cl_2$ |
| 18 | 2,3,4-$Cl_3$ |
| 19 | 2,3,5-$Cl_3$ |
| 11 | 2,3,6-$Cl_3$ |
| 12 | 2,4,5-$Cl_3$ |
| 13 | 2,4,6-$Cl_3$ |
| 14 | 3,4,5-$Cl_3$ |
| 15 | 2,3,4,6-$Cl_4$ |
| 16 | 2,3,5,6-$Cl_4$ |
| 17 | 2,3,4,5,6-$Cl_5$ |
| 18 | 2-Br |
| 19 | 3-Br |
| 20 | 4-Br |
| 21 | 2,4-$Br_2$ |
| 22 | 2,5-$Br_2$ |
| 23 | 2,6-$Br_2$ |
| 24 | 2,4,6-$Br_3$ |
| 25 | 2,3,4,5,6-$Br_5$ |
| 26 | 2-J |
| 27 | 3-J |
| 28 | 4-J |
| 29 | 2,4-$J_2$ |
| 30 | 2-Cl, 3-F |
| 31 | 2-Cl, 4-F |
| 32 | 2-Cl, 5-F |
| 33 | 2-Cl, 6-F |
| 34 | 2-Cl, 3-Br |
| 35 | 2-Cl, 4-Br |
| 36 | 2-Cl, 5-Br |
| 37 | 2-Cl, 6-Br |
| 38 | 2-Br, 3-Cl |
| 39 | 2-Br, 4-Cl |
| 40 | 2-Br, 5-Cl |
| 41 | 2-Br, 3-F |
| 42 | 2-Br, 4-F |
| 43 | 2-Br, 5-F |
| 44 | 2-Br, 6-F |
| 45 | 2-F, 3-Cl |

Tabelle A (fortgesetzt)

| Nummer | $R^x_p$ |
|---|---|
| 46 | 2-F, 4-Cl |
| 47 | 2-F, 5-Cl |
| 48 | 3-Cl, 4-F |
| 49 | 3-Cl, 5-F |
| 5C | 3-Cl, 4-Br |
| 51 | 3-Cl, 5-Br |
| 52 | 3-F, 4-Cl |
| 53 | 3-F, 4-Br |
| 54 | 3-Br, 4-Cl |
| 55 | 3-Br, 4-F |
| 56 | 2,6-$Cl_2$, 4-Br |
| 57 | 2-$CH_3$ |
| 58 | 3-$CH_3$ |
| 59 | 4-$CH_3$ |
| 60 | 2,3-$(CH_3)_2$ |
| 61 | 2,4-$(CH_3)_2$ |
| 62 | 2,5-$(CH_3)_2$ |
| 63 | 2,6-$(CH_3)_2$ |
| 64 | 3,4-$(CH_3)_2$ |
| 65 | 3,5-$(CH_3)_2$ |
| 66 | 2,3,5-$(CH_3)_3$ |
| 67 | 2,3,4-$(CH_3)_3$ |
| 68 | 2,3,6-$(CH_3)_3$ |
| 69 | 2,4,5-$(CH_3)_3$ |
| 70 | 2,4,6-$(CH_3)_3$ |
| 71 | 3,4,5-$(CH_3)_3$ |
| 72 | 2,3,4,6-$(CH_3)_4$ |
| 73 | 2,3,5,6-$(CH_3)_4$ |
| 74 | 2,3,4,5,6-$(CH_3)_5$ |
| 75 | 2-$C_2H_5$ |
| 76 | 3-$C_2H_5$ |
| 77 | 4-$C_2H_5$ |
| 78 | 2,4-$(C_2H_5)_5$ |
| 79 | 2,6-$(C_2H_5)_2$ |
| 80 | 3,5-$(C_2H_5)_2$ |
| 81 | 2,4,6-$(C_2H_5)_3$ |
| 82 | 2-n-$C_3H_7$ |
| 83 | 3-n-$C_3H_7$ |

Tabelle A (fortgesetzt)

| Nummer | $R^x_p$ |
|---|---|
| 84 | 4-n-$C_3H_7$ |
| 85 | 2-i-$C_3H_7$ |
| 86 | 3-i-$C_3H_7$ |
| 87 | 4-i-$C_3H_7$ |
| 88 | 2,4-(i-$C_3H_7)_2$ |
| 89 | 2,6-(i-$C_3H_7)_2$ |
| 90 | 3,5-(i-$C_3H_7)_2$ |
| 91 | 2-s-$C_4H_9$ |
| 92 | 3-s-$C_4H_9$ |
| 93 | 4-s-$C_4H_9$ |
| 94 | 2-t-$C_4H_9$ |
| 95 | 3-t-$C_4H_9$ |
| 96 | 4-t-$C_4H_9$ |
| 97 | 4-n-$C_9H_{19}$ |
| 98 | 2-$CH_3$, 4-t-$C_4H_9$ |
| 99 | 2-$CH_3$, 6-t-$C_4H_9$ |
| 100 | 2-$CH_3$, 4-i-$C_3H_7$ |
| 101 | 2-$CH_3$, 5-i-$C_3H_7$ |
| 102 | 3-$CH_3$, 4-i-$C_3H_7$ |
| 103 | 2-cyclo-$C_6H_{11}$ |
| 104 | 3-cyclo-$C_6H_{11}$ |
| 105 | 4-cyclo-$C_6H_{11}$ |
| 106 | 2-Cl, 4-$C_6H_5$ |
| 107 | 2-Br, 4-$C_6H_5$ |
| 108 | 2-$OCH_3$ |
| 109 | 3-$OCH_3$ |
| 110 | 4-$OCH_3$ |
| 111 | 2-$OC_2H_5$ |
| 112 | 3-O-$C_2H_5$ |
| 113 | 4-O-$C_2H_5$ |
| 114 | 2-O-n-$C_3H_7$ |
| 115 | 3-O-n-$C_3H_7$ |
| 116 | 4-O-n-$C_3H_7$ |
| 117 | 2-O-i-$C_3H_7$ |
| 118 | 3-O-i-$C_3H_7$ |
| 119 | 4-O-i-$C_3H_7$ |
| 120 | 2-O-n-$C_6H_{13}$ |
| 121 | 3-O-n-$C_6H_{13}$ |

Tabelle A (fortgesetzt)

| Nummer | $R^x_p$ |
|---|---|
| 122 | 4-O-n-$C_6H_{13}$ |
| 123 | 2-O-$CH_2C_6H_5$ |
| 124 | 3-O-$CH_2C_6H_5$ |
| 125 | 4-O-$CH_2C_6H_5$ |
| 126 | 2-O-$(CH_2)_3C_6H_5$ |
| 127 | 4-O-$(CH_2)_3C_6H_5$ |
| 128 | 2,3-$(OCH_3)_2$ |
| 129 | 2,4-$(OCH_3)_2$ |
| 130 | 2,5-$(OCH_3)_2$ |
| 131 | 2,6-$(OCH_3)_2$ |
| 132 | 3,4-$(OCH_3)_2$ |
| 133 | 3,5-$(OCH_3)_2$ |
| 134 | 2-O-t-$C_4H_9$ |
| 135 | 3-O-t-$C_4H_9$ |
| 136 | 4-O-t-$C_4H_9$ |
| 137 | 3-(3'-Cl-$C_6H_4$) |
| 138 | 4-(4'-$CH_3$-$C_6H_4$) |
| 139 | 2-O-$C_6H_5$ |
| 140 | 3-O-$C_6H_5$ |
| 141 | 4-O-$C_6H_5$ |
| 142 | 2-O-(2'-F-$C_6H_4$) |
| 143 | 3-O-(3'-Cl-$C_6H_4$) |
| 144 | 4-O-(4'-$CH_3$-$C_6H_4$) |
| 145 | 2,3,6-$(CH_3)_3$, 4-F |
| 146 | 2,3,6-$(CH_3)_3$, 4-Cl |
| 147 | 2,3,6-$(CH_3)_3$, 4-Br |
| 148 | 2,4-$(CH_3)_2$, 6-F |
| 149 | 2,4-$(CH_3)_2$, 6-Cl |
| 150 | 2,4-$(CH_3)_2$, 6-Br |
| 151 | 2-i-$C_3H_7$, 4-Cl, 5-$CH_3$ |
| 152 | 2-Cl, 4-$NO_2$ |
| 153 | 2-$NO_2$, 4-Cl |
| 154 | 2-$OCH_3$, 5-$NO_2$ |
| 155 | 2,4-$Cl_2$, 5-$NO_2$ |
| 156 | 2,4-$Cl_2$, 6-$NO_2$ |
| 157 | 2,6-$Cl_2$, 4-$NO_2$ |
| 158 | 2,6-$Br_2$, 4-$NO_2$ |
| 159 | 2,6-$J_2$, 4-$NO_2$ |

Tabelle A (fortgesetzt)

| Nummer | $R^x_p$ |
|---|---|
| 160 | 2-$CH_3$, 5-i-$C_3H_7$, 4-Cl |
| 161 | 2-$CO_2CH_3$ |
| 162 | 3-$CO_2CH_3$ |
| 163 | 4-$CO_2CH_3$ |
| 164 | 2-$CH_2$-$OCH_3$ |
| 165 | 3-$CH_2$-$OCH_3$ |
| 166 | 4-$CH_2$-$OCH_3$ |
| 167 | 2-Me-4-$CH_3$-CH($CH_3$)-CO |
| 168 | 2-$CH_3$-4-($CH_3$-C=NOC$H_3$) |
| 169 | 2-$CH_3$-4-($CH_3$-C=NO$C_2H_5$) |
| 170 | 2-$CH_3$-4-($CH_3$-C=NO-n-$C_3H_7$) |
| 171 | 2-$CH_3$-4-($CH_3$-C=NO-i-$C_3H_7$) |
| 172 | 2,5-($CH_3$)2-4-($CH_3$-C=NOC$H_3$) |
| 173 | 2,5-$(CH_3)_2$-4-($CH_3$-C=NO$C_2H_5$) |
| 174 | 2,5-($CH_3$-4-($CH_3$-C=NO-n-$C_3H_7$) |
| 175 | 2,5-$(CH_3)_2$-4-($CH_3$-C=NO-i-$C_3H_7$) |
| 176 | 2-$C_6H_5$ |
| 177 | 3-$C_6H_5$ |
| 178 | 4-$C_6H_5$ |
| 179 | 2-(2'-F-$C_6H_4$) |
| 180 | 2-$CH_3$, 5-Br |
| 181 | 2-$CH_3$, 6-Br |
| 182 | 2-Cl, 3-$CH_3$ |
| 183 | 2-Cl, 4-$CH_3$ |
| 184 | 2-Cl, 5-$CH_3$ |
| 185 | 2-F, 3-$CH_3$ |
| 186 | 2-F, 4-$CH_3$ |
| 187 | 2-F, 5-$CH_3$ |
| 188 | 2-Br, 3-$CH_3$ |
| 189 | 2-Br, 4-$CH_3$ |
| 190 | 2-Br, 5-$CH_3$ |
| 191 | 3-$CH_3$, 4-Cl |
| 192 | 3-$CH_3$, 5-Cl |
| 193 | 3-$CH_3$, 4-F |
| 194 | 3-$CH_3$, 5-F |
| 195 | 3-$CH_3$, 4-Br |
| 196 | 3-$CH_3$, 5-Br |
| 197 | 3-F, 4-$CH_3$ |

Tabelle A (fortgesetzt)

| Nummer | $R^x_p$ |
|---|---|
| 198 | 3-Cl, 4-$CH_3$ |
| 199 | 3-Br, 4-$CH_3$ |
| 200 | 2-Cl, 4,5-$(CH_3)_2$ |
| 201 | 2-Br, 4,5-$(CH_3)_2$ |
| 292 | 2-Cl, 3,5-$(CH_3)_2$ |
| 203 | 2-Br, 3,5-$(CH_3)_2$ |
| 204 | 2,6-$Cl_2$, 4-$CH_3$ |
| 205 | 2,6-$F_2$, 4-$CH_3$ |
| 206 | 2,6-$Br_2$, 4-$CH_3$ |
| 207 | 2,4-$Br_2$, 6-$CH_3$ |
| 208 | 2,4-$F_2$, 6-$CH_3$ |
| 209 | 2,4-$Br_2$, 6-$CH_3$ |
| 210 | 2,6-$(CH_3)_2$, 4-F |
| 211 | 2,6-$(CH_3)_2$, 4-Cl |
| 212 | 2,6-$(CH_3)_2$, 4-Br |
| 213 | 3,5-$(CH_3)_2$, 4-F |
| 214 | 3,5-$(CH_3)_2$, 4-Cl |
| 215 | 3,5-$(CH_3)_2$, 4-Br |
| 216 | 2-$CF_3$ |
| 217 | 3-$CF_3$ |
| 218 | 4-$CF_3$ |
| 219 | 2-$OCF_3$ |
| 220 | 3-$OCF_3$ |
| 221 | 4-$OCF_3$ |
| 222 | 3-$OCH_2CHF_2$ |
| 223 | 2-$NO_2$ |
| 224 | 3-$NO_2$ |
| 225 | 4-$NO_2$ |
| 226 | 2-CN |
| 227 | 3-CN |
| 228 | 4-CN |
| 229 | 2-$CH_3$, 3-Cl |
| 230 | 2-$CH_3$, 4-Cl |
| 231 | 2-$CH_3$, 5-Cl |
| 232 | 2-$CH_3$, 6-Cl |
| 233 | 2-$CH_3$, 3-F |
| 234 | 2-$CH_3$, 4-F |
| 235 | 2-$CH_3$, 5-F |

Tabelle A (fortgesetzt)

| Nummer | $R^x_p$ |
|---|---|
| 236 | 2-$CH_3$, 6-F |
| 237 | 2-$CH_3$, 3-Br |
| 238 | 2-$CH_3$, 4-Br |
| 239 | 2-Pyridyl-2' |
| 240 | 3-Pyridyl-3' |
| 241 | 4-Pyridyl-4' |

Tabelle B

| Nummer | $R^1$ | $R^y$ | $R^z$ | $R^3$ | $R^4$ |
|---|---|---|---|---|---|
| 334 | H | H | H | Cyclohexyl | $CH_3$ |
| 335 | H | H | H | Benzyl | $CH_3$ |
| 336 | H | H | H | 2-Pyridyl | $CH_3$ |
| 337 | H | H | H | 5-Cl-pyridyl-2 | $CH_3$ |
| 338 | H | H | H | 5-$CF_3$-pyridyl -2 | $CH_3$ |
| 339 | H | H | H | 2-Pyrazinyl | $CH_3$ |
| 340 | H | H | Cl | Cyclohexyl | $CH_3$ |
| 341 | H | H | Cl | Benzyl | $CH_3$ |
| 342 | H | H | Cl | 2-Pyridyl | $CH_3$ |
| 343 | H | H | Cl | 5-Cl-pyridyl-2 | $CH_3$ |
| 344 | H | H | Cl | 5-$CF_3$-pyridyl -2 | $CH_3$ |
| 345 | H | H | Cl | 2-Pyrazinyl | $CH_3$ |
| 346 | H | $CH_3$ | H | Cyclohexyl | $CH_3$ |
| 347 | H | $CH_3$ | H | Benzyl | $CH_3$ |
| 348 | H | $CH_3$ | H | 2-Pyridyl | $CH_3$ |
| 349 | H | $CH_3$ | H | 5-Cl-pyridyl-2 | $CH_3$ |
| 350 | H | $CH_3$ | H | 5-$CF_3$-pyridyl -2 | $CH_3$ |
| 351 | H | $CH_3$ | H | 2-Pyrazinyl | $CH_3$ |
| 352 | H | H | H | Cyclohexyl | $C_2H_5$ |
| 353 | H | H | H | Benzyl | $C_2H_5$ |
| 354 | H | H | H | Phenyl | $C_2H_5$ |
| 355 | H | H | H | 2-Pyridyl | $C_2H_5$ |
| 356 | H | H | H | 5-Cl-pyridyl-2 | $C_2H_5$ |
| 357 | H | H | H | 5-$CF_3$-pyridyl -2 | $C_2H_5$ |
| 358 | H | H | H | 2-Pyrazinyl | $C_2H_5$ |
| 359 | H | H | Cl | Cyclohexyl | $C_2H_5$ |
| 360 | H | H | Cl | Benzyl | $C_2H_5$ |
| 361 | H | H | Cl | Phenyl | $C_2H_5$ |
| 362 | H | H | Cl | 2-Pyridyl | $C_2H_5$ |

Tabelle B (fortgesetzt)

| Nummer | $R^1$ | $R^y$ | $R^z$ | $R^3$ | $R^4$ |
|---|---|---|---|---|---|
| 363 | H | H | Cl | 5-Cl-pyridyl-2 | $C_2H_5$ |
| 364 | H | H | Cl | 5-$CF_3$-pyridyl -2 | $C_2H_5$ |
| 365 | H | H | Cl | 2-Pyrazinyl | $C_2H_5$ |
| 366 | H | $CH_3$ | H | Cyclohexyl | $C_2H_5$ |
| 367 | H | $CH_3$ | H | Benzyl | $C_2H_5$ |
| 368 | H | $CH_3$ | H | Phenyl | $C_2H_5$ |
| 369 | H | $CH_3$ | H | 2-Pyridyl | $C_2H_5$ |
| 370 | H | $CH_3$ | H | 5-Cl-pyridyl-2 | $C_2H_5$ |
| 371 | H | $CH_3$ | H | 5-$CF_3$-pyridyl -2 | $C_2H_5$ |
| 372 | H | $CH_3$ | H | 2-Pyrazinyl | $C_2H_5$ |
| 373 | H | H | H | Cyclohexyl | $CH_2OCH_3$ |
| 374 | H | H | H | Benzyl | $CH_2OCH_3$ |
| 375 | H | H | H | Phenyl | $CH_2OCH_3$ |
| 376 | H | H | H | 2-Pyridyl | $CH_2OCH_3$ |
| 377 | H | H | H | 5-Cl-pyridyl-2 | $CH_2OCH_3$ |
| 378 | H | H | H | 5-$CF_3$-pyridyl -2 | $CH_2OCH_3$ |
| 379 | H | H | H | 2-Pyrazinyl | $CH_2OCH_3$ |
| 380 | H | H | Cl | Cyclohexyl | $CH_2OCH_3$ |
| 381 | H | H | Cl | Benzyl | $CH_2OCH_3$ |
| 382 | H | H | Cl | Phenyl | $CH_2OCH_3$ |
| 383 | H | H | Cl | 2-Pyridyl | $CH_2OCH_3$ |
| 384 | H | H | Cl | 5-Cl-pyridyl-2 | $CH_2OCH_3$ |
| 385 | H | H | Cl | 5-$CF_3$-pyridyl -2 | $CH_2OCH_3$ |
| 386 | H | H | Cl | 2-Pyrazinyl | $CH_2OCH_3$ |
| 387 | H | $CH_3$ | H | Cyclohexyl | $CH_2OCH_3$ |
| 388 | H | $CH_3$ | H | Benzyl | $CH_2OCH_3$ |
| 389 | H | $CH_3$ | H | Phenyl | $CH_2OCH_3$ |
| 390 | H | $CH_3$ | H | 2-Pyridyl | $CH_2OCH_3$ |
| 391 | H | $CH_3$ | H | 5-Cl-pyridyl-2 | $CH_2OCH_3$ |
| 392 | H | $CH_3$ | H | 5-$CF_3$-pyridyl -2 | $CH_2OCH_3$ |
| 393 | H | $CH_3$ | H | 2-Pyrazinyl | $CH_2OCH_3$ |
| 394 | H | H | H | Cyclohexyl | $CH_2C{\equiv}CH$ |
| 395 | H | H | H | Benzyl | $CH_2C{\equiv}CH$ |
| 396 | H | H | H | Phenyl | $CH_2C{\equiv}CH$ |
| 397 | H | H | H | 2-Pyridyl | $CH_2C{\equiv}CH$ |
| 398 | H | H | H | 5-Cl-pyridyl-2 | $CH_2C{\equiv}CH$ |
| 399 | H | H | H | 5-$CF_3$-pyridyl -2 | $CH_2C{\equiv}CH$ |
| 400 | H | H | H | 2-Pyrazinyl | $CH_2C{\equiv}CH$ |

Tabelle B (fortgesetzt)

| Nummer | $R^1$ | $R^y$ | $R^z$ | $R^3$ | $R^4$ |
|---|---|---|---|---|---|
| 401 | H | H | Cl | Cyclohexyl | $CH_2C{\equiv}CH$ |
| 402 | H | H | Cl | Benzyl | $CH_2C{\equiv}CH$ |
| 403 | H | H | Cl | Phenyl | $CH_2C{\equiv}CH$ |
| 404 | H | H | Cl | 2-Pyridyl | $CH_2C{\equiv}CH$ |
| 405 | H | H | Cl | 5-Cl-pyridyl-2 | $CH_2C{\equiv}CH$ |
| 406 | H | H | Cl | 5-$CF_3$-pyridyl -2 | $CH_2C{\equiv}CH$ |
| 407 | H | H | Cl | 2-Pyrazinyl | $CH_2C{\equiv}CH$ |
| 408 | H | $CH_3$ | H | Cyclohexyl | $CH_2C{\equiv}CH$ |
| 409 | H | $CH_3$ | H | Benzyl | $CH_2C{\equiv}CH$ |
| 410 | H | $CH_3$ | H | Phenyl | $CH_2C{\equiv}CH$ |
| 411 | H | $CH_3$ | H | 2-Pyridyl | $CH_2C{\equiv}CH$ |
| 412 | H | $CH_3$ | H | 5-Cl-pyridyl-2 | $CH_2C{\equiv}CH$ |
| 413 | H | $CH_3$ | H | 5-$CF_3$-pyridyl -2 | $CH_2C{\equiv}CH$ |
| 414 | H | $CH_3$ | H | 2-Pyrazinyl | $CH_2C{\equiv}CH$ |
| 415 | 3-F | H | H | Cyclohexyl | $CH_3$ |
| 416 | 3-F | H | H | Benzyl | $CH_3$ |
| 417 | 3-F | H | H | Phenyl | $CH_3$ |
| 418 | 3-F | H | H | 2-Pyridyl | $CH_3$ |
| 419 | 3-F | H | H | 5-Cl-pyridyl-2 | $CH_3$ |
| 420 | 3-F | H | H | 5-$CF_3$-pyridyl -2 | $CH_3$ |
| 421 | 3-F | H | H | 2-Pyrazinyl | $CH_3$ |
| 422 | 3-F | H | Cl | Cyclohexyl | $CH_3$ |
| 423 | 3-F | H | Cl | Benzyl | $CH_3$ |
| 424 | 3-F | H | Cl | Phenyl | $CH_3$ |
| 425 | 3-F | H | Cl | 2-Pyridyl | $CH_3$ |
| 426 | 3-F | H | Cl | 5-Cl-pyridyl-2 | $CH_3$ |
| 427 | 3-F | H | Cl | 5-$CF_3$-pyridyl -2 | $CH_3$ |
| 428 | 3-F | H | Cl | 2-Pyrazinyl | $CH_3$ |
| 429 | 3-F | $CH_3$ | H | Cyclohexyl | $CH_3$ |
| 430 | 3-F | $CH_3$ | H | Benzyl | $CH_3$ |
| 431 | 3-F | $CH_3$ | H | Phenyl | $CH_3$ |
| 432 | 3-F | $CH_3$ | H | 2-Pyridyl | $CH_3$ |
| 433 | 3-F | $CH_3$ | H | 5-Cl-pyridyl-2 | $CH_3$ |
| 434 | 3-F | $CH_3$ | H | 5-$CF_3$-pyridyl -2 | $CH_3$ |
| 435 | 3-F | $CH_3$ | H | 2-Pyrazinyl | $CH_3$ |
| 436 | 3-F | H | H | Cyclohexyl | $C_2H_5$ |
| 437 | 3-F | H | H | Benzyl | $C_2H_5$ |
| 438 | 3-F | H | H | Phenyl | $C_2H_5$ |

Tabelle B (fortgesetzt)

| Nummer | $R^1$ | $R^y$ | $R^z$ | $R^3$ | $R^4$ |
|---|---|---|---|---|---|
| 439 | 3-F | H | H | 2-Pyridyl | $C_2H_5$ |
| 440 | 3-F | H | H | 5-Cl-pyridyl-2 | $C_2H_5$ |
| 441 | 3-F | H | H | 5-$CF_3$-pyridyl -2 | $C_2H_5$ |
| 442 | 3-F | H | H | 2-Pyrazinyl | $C_2H_5$ |
| 443 | 3-F | H | Cl | Cyclohexyl | $C_2H_5$ |
| 444 | 3-F | H | Cl | Benzyl | $C_2H_5$ |
| 445 | 3-F | H | Cl | Phenyl | $C_2H_5$ |
| 446 | 3-F | H | Cl | 2-Pyridyl | $C_2H_5$ |
| 447 | 3-F | H | Cl | 5-Cl-pyridyl-2 | $C_2H_5$ |
| 448 | 3-F | H | Cl | 5-$CF_3$-pyridyl -2 | $C_2H_5$ |
| 449 | 3-F | H | Cl | 2-Pyrazinyl | $C_2H_5$ |
| 450 | 3-F | H | H | Cyclohexyl | $CH_2OCH_3$ |
| 451 | 3-F | H | H | Benzyl | $CH_2OCH_3$ |
| 452 | 3-F | H | H | Phenyl | $CH_2OCH_3$ |
| 453 | 3-F | H | H | 2-Pyridyl | $CH_2OCH_3$ |
| 454 | 3-F | H | H | 5-Cl-pyridyl-2 | $CH_2OCH_3$ |
| 455 | 3-F | H | H | 5-$CF_3$-pyridyl -2 | $CH_2OCH_3$ |
| 456 | 3-F | H | H | 2-Pyrazinyl | $CH_2OCH_3$ |
| 457 | 3-F | H | Cl | Cyclohexyl | $CH_2OCH_3$ |
| 458 | 3-F | H | Cl | Benzyl | $CH_2OCH_3$ |
| 459 | 3-F | H | Cl | Phenyl | $CH_2OCH_3$ |
| 460 | 3-F | H | Cl | 2-Pyridyl | $CH_2OCH_3$ |
| 461 | 3-F | H | Cl | 5-Cl-pyridyl-2 | $CH_2OCH_3$ |
| 462 | 3-F | H | Cl | 5-$CF_3$-pyridyl -2 | $CH_2OCH_3$ |
| 463 | 3-F | H | Cl | 2-Pyrazinyl | $CH_2OCH_3$ |
| 464 | 3-F | $CH_3$ | H | Cyclohexyl | $CH_2OCH_3$ |
| 465 | 3-F | $CH_3$ | H | Benzyl | $CH_2OCH_3$ |
| 466 | 3-F | $CH_3$ | H | Phenyl | $CH_2OCH_3$ |
| 467 | 3-F | $CH_3$ | H | 2-Pyridyl | $CH_2OCH_3$ |
| 468 | 3-F | $CH_3$ | H | 5-Cl-pyridyl-2 | $CH_2OCH_3$ |
| 469 | 3-F | $CH_3$ | H | 5-$CF_3$-pyridyl -2 | $CH_2OCH_3$ |
| 470 | 3-F | $CH_3$ | H | 2-Pyrazinyl | $CH_2OCH_3$ |
| 471 | 3-F | H | H | Cyclohexyl | $CH_2C{\equiv}CH$ |
| 472 | 3-F | H | H | Benzyl | $CH_2C{\equiv}CH$ |
| 473 | 3-F | H | H | Phenyl | $CH_2C{\equiv}CH$ |
| 474 | 3-F | H | H | 2-Pyridyl | $CH_2C{\equiv}CH$ |
| 475 | 3-F | H | H | 5-Cl-pyridyl-2 | $CH_2C{\equiv}CH$ |
| 476 | 3-F | H | H | 5-$CF_3$-pyridyl -2 | $CH_2C{\equiv}CH$ |

Tabelle B (fortgesetzt)

| Nummer | $R^1$ | $R^y$ | $R^z$ | $R^3$ | $R^4$ |
|---|---|---|---|---|---|
| 477 | 3-F | H | H | 2-Pyrazinyl | $CH_2C{\equiv}CH$ |
| 478 | 3-F | H | Cl | Cyclohexyl | $CH_2C{\equiv}CH$ |
| 479 | 3-F | H | Cl | Benzyl | $CH_2C{\equiv}CH$ |
| 480 | 3-F | H | Cl | Phenyl | $CH_2C{\equiv}CH$ |
| 481 | 3-F | H | Cl | 2-Pyridyl | $CH_2C{\equiv}CH$ |
| 482 | 3-F | H | Cl | 5-Cl-pyridyl-2 | $CH_2C{\equiv}CH$ |
| 483 | 3-F | H | Cl | 5-$CF_3$-pyridyl -2 | $CH_2C{\equiv}CH$ |
| 484 | 3-F | H | Cl | 2-Pyrazinyl | $CH_2C{\equiv}CH$ |
| 485 | 3-F | $CH_3$ | H | Cyclohexyl | $CH_2C{\equiv}CH$ |
| 486 | 3-F | $CH_3$ | H | Benzyl | $CH_2C{\equiv}CH$ |
| 487 | 3-F | $CH_3$ | H | Phenyl | $CH_2C{\equiv}CH$ |
| 488 | 3-F | $CH_3$ | H | 2-Pyridyl | $CH_2C{\equiv}CH$ |
| 489 | 3-F | $CH_3$ | H | 5-Cl-pyridyl-2 | $CH_2C{\equiv}CH$ |
| 490 | 3-F | $CH_3$ | H | 5-$CF_3$-pyridyl -2 | $CH_2C{\equiv}CH$ |
| 491 | 3-F | $CH_3$ | H | 2-Pyrazinyl | $CH_2C{\equiv}CH$ |
| 492 | 6-Cl | H | H | Cyclohexyl | $CH_3$ |
| 493 | 6-Cl | H | H | Benzyl | $CH_3$ |
| 494 | 6-Cl | H | H | Phenyl | $CH_3$ |
| 495 | 6-Cl | H | H | 2-Pyridyl | $CH_3$ |
| 496 | 6-Cl | H | H | 5-Cl-pyridyl-2 | $CH_3$ |
| 497 | 6-Cl | H | H | 5-$CF_3$-pyridyl -2 | $CH_3$ |
| 498 | 6-Cl | H | H | 2-Pyrazinyl | $CH_3$ |
| 499 | 6-Cl | H | Cl | Cyclohexyl | $CH_3$ |
| 500 | 6-Cl | H | Cl | Benzyl | $CH_3$ |
| 501 | 6-Cl | H | Cl | Phenyl | $CH_3$ |
| 502 | 6-Cl | H | Cl | 2-Pyridyl | $CH_3$ |
| 503 | 6-Cl | H | Cl | 5-Cl-pyridyl-2 | $CH_3$ |
| 504 | 6-Cl | H | Cl | 5-$CF_3$-pyridyl -2 | $CH_3$ |
| 505 | 6-Cl | H | Cl | 2-Pyrazinyl | $CH_3$ |
| 506 | 6-Cl | $CH_3$ | H | Cyclohexyl | $CH_3$ |
| 507 | 6-Cl | $CH_3$ | H | Benzyl | $CH_3$ |
| 508 | 6-C1 | $CH_3$ | H | Phenyl | $CH_3$ |
| 509 | 6-Cl | $CH_3$ | H | 2-Pyridyl | $CH_3$ |
| 510 | 6-Cl | $CH_3$ | H | 5-Cl-pyridyl-2 | $CH_3$ |
| 511 | 6-Cl | $CH_3$ | H | 5-$CF_3$-pyridyl -2 | $CH_3$ |
| 512 | 6-Cl | $CH_3$ | H | 2-Pyrazinyl | $CH_3$ |
| 513 | 6-Cl | H | H | Cyclohexyl | $C_2H_5$ |
| 514 | 6-Cl | H | H | Benzyl | $C_2H_5$ |

Tabelle B (fortgesetzt)

| Nummer | $R^1$ | $R^y$ | $R^z$ | $R^3$ | $R^4$ |
|---|---|---|---|---|---|
| 515 | 6-Cl | H | H | Phenyl | $C_2H_5$ |
| 516 | 6-Cl | H | H | 2-Pyridyl | $C_2H_5$ |
| 517 | 6-Cl | H | H | 5-Cl-pyridyl-2 | $C_2H_5$ |
| 518 | 6-Cl | H | H | 5-$CF_3$-pyridyl -2 | $C_2H_5$ |
| 519 | 6-Cl | H | H | 2-Pyrazinyl | $C_2H_5$ |
| 520 | 6-Cl | H | Cl | Cyclohexyl | $C_2H_5$ |
| 521 | 6-Cl | H | Cl | Benzyl | $C_2H_5$ |
| 522 | 6-Cl | H | Cl | Phenyl | $C_2H_5$ |
| 523 | 6-Cl | H | Cl | 2-Pyridyl | $C_2H_5$ |
| 524 | 6-Cl | H | Cl | 5-Cl-pyridyl-2 | $C_2H_5$ |
| 525 | 6-Cl | H | Cl | 5-$CF_3$-pyridyl -2 | $C_2H_5$ |
| 526 | 6-Cl | H | Cl | 2-Pyrazinyl | $C_2H_5$ |
| 527 | 6-Cl | $CH_3$ | H | Cyclohexyl | $C_2H_5$ |
| 528 | 6-Cl | $CH_3$ | H | Benzyl | $C_2H_5$ |
| 529 | 6-Cl | $CH_3$ | H | Phenyl | $C_2H_5$ |
| 530 | 6-Cl | $CH_3$ | H | 2-Pyridyl | $C_2H_5$ |
| 531 | 6-Cl | $CH_3$ | H | 5-Cl-pyridyl-2 | $C_2H_5$ |
| 532 | 6-Cl | $CH_3$ | H | 5-$CF_3$-pyridyl -2 | $C_2H_5$ |
| 533 | 6-Cl | $CH_3$ | H | 2-Pyrazinyl | $C_2H_5$ |
| 534 | 6-Cl | H | H | Cyclohexyl | $CH_2OCH_3$ |
| 535 | 6-Cl | H | H | Benzyl | $CH_2OCH_3$ |
| 536 | 6-Cl | H | H | Phenyl | $CH_2OCH_3$ |
| 537 | 6-Cl | H | H | 2-Pyridyl | $CH_2OCH_3$ |
| 538 | 6-Cl | H | H | 5-Cl-pyridyl-2 | $CH_2OCH_3$ |
| 539 | 6-Cl | H | H | 5-$CF_3$-pyridyl -2 | $CH_2OCH_3$ |
| 540 | 6-Cl | H | H | 2-Pyrazinyl | $CH_2OCH_3$ |
| 541 | 6-Cl | H | Cl | Cyclohexyl | $CH_2OCH_3$ |
| 542 | 6-Cl | H | Cl | Benzyl | $CH_2OCH_3$ |
| 543 | 6-Cl | H | Cl | Phenyl | $CH_2OCH_3$ |
| 544 | 6-Cl | H | Cl | 2-Pyridyl | $CH_2OCH_3$ |
| 545 | 6-Cl | H | Cl | 5-Cl-pyridyl-2 | $CH_2OCH_3$ |
| 546 | 6-Cl | H | Cl | 5-$CF_3$-pyridyl -2 | $CH_2OCH_3$ |
| 547 | 6-Cl | H | Cl | 2-Pyrazinyl | $CH_2OCH_3$ |
| 548 | 6-Cl | $CH_3$ | H | Cyclohexyl | $CH_2OCH_3$ |
| 549 | 6-Cl | $CH_3$ | H | Benzyl | $CH_2OCH_3$ |
| 550 | 6-Cl | $CH_3$ | H | Phenyl | $CH_2OCH_3$ |
| 551 | 6-Cl | $CH_3$ | H | 2-Pyridyl | $CH_2OCH_3$ |
| 552 | 6-Cl | $CH_3$ | H | 5-Cl-pyridyl-2 | $CH_2OCH_3$ |

Tabelle B (fortgesetzt)

| Nummer | $R^1$ | $R^y$ | $R^z$ | $R^3$ | $R^4$ |
|---|---|---|---|---|---|
| 553 | 6-Cl | $CH_3$ | H | 5-$CF_3$-pyridyl -2 | $CH_2OCH_3$ |
| 554 | 6-Cl | $CH_3$ | H | 2-Pyrazinyl | $CH_2OCH_3$ |
| 555 | 6-Cl | H | H | Cyclohexyl | $CH_2C{\equiv}CH$ |
| 556 | 6-Cl | H | H | Benzyl | $CH_2C{\equiv}CH$ |
| 557 | 6-Cl | H | H | Phenyl | $CH_2C{\equiv}CH$ |
| 558 | 6-Cl | H | H | 2-Pyridyl | $CH_2C{=}CH$ |
| 559 | 6-Cl | H | H | 5-Cl-pyridyl-2 | $CH_2C{\equiv}CH$ |
| 560 | 6-Cl | H | H | 5-$CF_3$-pyridyl -2 | $CH_2C{\equiv}CH$ |
| 561 | 6-Cl | H | H | 2-Pyrazinyl | $CH_2C{\equiv}CH$ |
| 562 | 6-Cl | H | Cl | Cyclohexyl | $CH_2C{\equiv}CH$ |
| 563 | 6-Cl | H | Cl | Benzyl | $CH_2C{\equiv}CH$ |
| 564 | 6-Cl | H | Cl | Phenyl | $CH_2C{\equiv}CH$ |
| 565 | 6-Cl | H | Cl | 2-Pyridyl | $CH_2C{\equiv}CH$ |
| 566 | 6-Cl | H | Cl | 5-Cl-pyridyl-2 | $CH_2C{\equiv}CH$ |
| 567 | 6-Cl | H | Cl | 5-$CF_3$-pyridyl -2 | $CH_2C{\equiv}CH$ |
| 568 | 6-Cl | H | Cl | 2-Pyrazinyl | $CH_2C{\equiv}CH$ |
| 569 | 6-Cl | $CH_3$ | H | Cyclohexyl | $CH_2C{\equiv}CH$ |
| 570 | 6-Cl | $CH_3$ | H | Benzyl | $CH_2C{\equiv}CH$ |
| 571 | 6-Cl | $CH_3$ | H | Phenyl | $CH_2C{\equiv}CH$ |
| 572 | 6-Cl | $CH_3$ | H | 2-Pyridyl | $CH_2C{\equiv}CH$ |
| 573 | 6-Cl | $CH_3$ | H | 5-Cl-pyridyl-2 | $CH_2C{\equiv}CH$ |
| 574 | 6-Cl | $CH_3$ | H | 5-$CF_3$-pyridyl -2 | $CH_2C{\equiv}CH$ |
| 575 | 6-Cl | $CH_3$ | H | 2-Pyrazinyl | $CH_2C{\equiv}CH$ |
| 576 | 6-$CH_3$ | H | H | Cyclohexyl | $CH_3$ |
| 577 | 6-$CH_3$ | H | H | Benzyl | $CH_3$ |
| 578 | 6-$CH_3$ | H | H | Phenyl | $CH_3$ |
| 579 | 6-$CH_3$ | H | H | 2-Pyridyl | $CH_3$ |
| 580 | 6-$CH_3$ | H | H | 5-Cl-pyridyl-2 | $CH_3$ |
| 581 | 6-$CH_3$ | H | H | 5-$CF_3$-pyridyl -2 | $CH_3$ |
| 582 | 6-$CH_3$ | H | H | 2-Pyrazinyl | $CH_3$ |
| 583 | 6-$CH_3$ | H | Cl | Cyclohexyl | $CH_3$ |
| 584 | 6-$CH_3$ | H | Cl | Benzyl | $CH_3$ |
| 585 | 6-$CH_3$ | H | Cl | Phenyl | $CH_3$ |
| 586 | 6-$CH_3$ | H | Cl | 2-Pyridyl | $CH_3$ |
| 587 | 6-$CH_3$ | H | Cl | 5-Cl-pyridyl-2 | $CH_3$ |
| 588 | 6-$CH_3$ | H | Cl | 5-$CF_3$-pyridyl -2 | $CH_3$ |
| 589 | 6-$CH_3$ | H | Cl | 2-Pyrazinyl | $CH_3$ |
| 590 | 6-$CH_3$ | $CH_3$ | H | Cyclohexyl | $CH_3$ |

Tabelle B (fortgesetzt)

| Nummer | $R^1$ | $R^y$ | $R^z$ | $R^3$ | $R^4$ |
|---|---|---|---|---|---|
| 591 | 6-$CH_3$ | $CH_3$ | H | Benzyl | $CH_3$ |
| 592 | 6-$CH_3$ | $CH_3$ | H | Phenyl | $CH_3$ |
| 593 | 6-$CH_3$ | $CH_3$ | H | 2-Pyridyl | $CH_3$ |
| 594 | 6-$CH_3$ | $CH_3$ | H | 5-Cl-pyridyl-2 | $CH_3$ |
| 595 | 6-$CH_3$ | $CH_3$ | H | 5-$CF_3$-pyridyl -2 | $CH_3$ |
| 596 | 6-$CH_3$ | $CH_3$ | H | 2-Pyrazinyl | $CH_3$ |
| 597 | 6-$CH_3$ | H | H | Cyclohexyl | $C_2H_5$ |
| 598 | 6-$CH_3$ | H | H | Benzyl | $C_2H_5$ |
| 599 | 6-$CH_3$ | H | H | Phenyl | $C_2H_5$ |
| 600 | 6-$CH_3$ | H | H | 2-Pyridyl | $C_2H_5$ |
| 601 | 6-$CH_3$ | H | H | 5-Cl-pyridyl-2 | $C_2H_5$ |
| 602 | 6-$CH_3$ | H | H | 5-$CF_3$-pyridyl -2 | $C_2H_5$ |
| 603 | 6-$CH_3$ | H | H | 2-Pyrazinyl | $C_2H_5$ |
| 604 | 6-$CH_3$ | H | Cl | Cyclohexyl | $C_2H_5$ |
| 605 | 6-$CH_3$ | H | Cl | Benzyl | $C_2H_5$ |
| 606 | 6-$CH_3$ | H | Cl | Phenyl | $C_2H_5$ |
| 607 | 6-$CH_3$ | H | Cl | 2-Pyridyl | $C_2H_5$ |
| 608 | 6-$CH_3$ | H | Cl | 5-Cl-pyridyl-2 | $C_2H_5$ |
| 609 | 6-$CH_3$ | H | Cl | 5-$CF_3$-pyridyl -2 | $C_2H_5$ |
| 610 | 6-$CH_3$ | H | Cl | 2-Pyrazinyl | $C_2H_5$ |
| 611 | 6-$CH_3$ | $CH_3$ | H | Cyclohexyl | $C_2H_5$ |
| 612 | 6-$CH_3$ | $CH_3$ | H | Benzyl | $C_2H_5$ |
| 613 | 6-$CH_3$ | $CH_3$ | H | Phenyl | $C_2H_5$ |
| 614 | 6-$CH_3$ | $CH_3$ | H | 2-Pyridyl | $C_2H_5$ |
| 615 | 6-$CH_3$ | $CH_3$ | H | 5-Cl-pyridyl-2 | $C_2H_5$ |
| 616 | 6-$CH_3$ | $CH_3$ | H | 5-$CF_3$-pyridyl -2 | $C_2H_5$ |
| 617 | 6-$CH_3$ | $CH_3$ | H | 2-Pyrazinyl | $C_2H_5$ |
| 618 | 6-$CH_3$ | H | H | Cyclohexyl | $CH_2OCH_3$ |
| 619 | 6-$CH_3$ | H | H | Benzyl | $CH_2OCH_3$ |
| 620 | 6-$CH_3$ | H | H | Phenyl | $CH_2OCH_3$ |
| 621 | 6-$CH_3$ | H | H | 2-Pyridyl | $CH_2OCH_3$ |
| 622 | 6-$CH_3$ | H | H | 5-Cl-pyridyl-2 | $CH_2OCH_3$ |
| 623 | 6-$CH_3$ | H | H | 5-$CF_3$-pyridyl -2 | $CH_2OCH_3$ |
| 624 | 6-$CH_3$ | H | H | 2-Pyrazinyl | $CH_2OCH_3$ |
| 625 | 6-$CH_3$ | H | Cl | Cyclohexyl | $CH_2OCH_3$ |
| 626 | 6-$CH_3$ | H | Cl | Benzyl | $CH_2OCH_3$ |
| 627 | 6-$CH_3$ | H | Cl | Phenyl | $CH_2OCH_3$ |
| 628 | 6-$CH_3$ | H | Cl | 2-Pyridyl | $CH_2OCH_3$ |

Tabelle B (fortgesetzt)

| Nummer | $R^1$ | $R^y$ | $R^z$ | $R^3$ | $R^4$ |
|---|---|---|---|---|---|
| 629 | 6-$CH_3$ | H | Cl | 5-Cl-pyridyl-2 | $CH_2OCH_3$ |
| 630 | 6-$CH_3$ | H | Cl | 5-$CF_3$-pyridyl -2 | $CH_2OCH_3$ |
| 631 | 6-$CH_3$ | H | Cl | 2-Pyrazinyl | $CH_2OCH_3$ |
| 632 | 6-$CH_3$ | $CH_3$ | H | Cyclohexyl | $CH_2OCH_3$ |
| 633 | 6-$CH_3$ | $CH_3$ | H | Benzyl | $CH_2OCH_3$ |
| 634 | 6-$CH_3$ | $CH_3$ | H | Phenyl | $CH_2OCH_3$ |
| 635 | 6-$CH_3$ | $CH_3$ | H | 2-Pyridyl | $CH_2OCH_3$ |
| 636 | 6-$CH_3$ | $CH_3$ | H | 5-Cl-pyridyl-2 | $CH_2OCH_3$ |
| 637 | 6-$CH_3$ | $CH_3$ | H | 5-$CF_3$-pyridyl -2 | $CH_2OCH_3$ |
| 638 | 6-$CH_3$ | $CH_3$ | H | 2-Pyrazinyl | $CH_2OCH_3$ |
| 639 | 6-$CH_3$ | H | H | Cyclohexyl | $CH_2C{\equiv}CH$ |
| 640 | 6-$CH_3$ | H | H | Benzyl | $CH_2C{\equiv}CH$ |
| 641 | 6-$CH_3$ | H | H | Phenyl | $CH_2C{\equiv}CH$ |
| 642 | 6-$CH_3$ | H | H | 2-Pyridyl | $CH_2C{\equiv}CH$ |
| 643 | 6-$CH_3$ | H | H | 5-Cl-pyridyl-2 | $CH_2C{\equiv}CH$ |
| 644 | 6-$CH_3$ | H | H | 5-$CF_3$-pyridyl -2 | $CH_2C{\equiv}CH$ |
| 645 | 6-$CH_3$ | H | H | 2-Pyrazinyl | $CH_2C{\equiv}CH$ |
| 646 | 6-$CH_3$ | H | Cl | Cyclohexyl | $CH_2C{\equiv}CH$ |
| 647 | 6-$CH_3$ | H | Cl | Benzyl | $CH_2C{\equiv}CH$ |
| 648 | 6-$CH_3$ | H | Cl | Phenyl | $CH_2C{\equiv}CH$ |
| 649 | 6-$CH_3$ | H | Cl | 2-Pyridyl | $CH_2C{\equiv}CH$ |
| 650 | 6-$CH_3$ | H | Cl | 5-Cl-pyridyl-2 | $CH_2C{\equiv}CH$ |
| 651 | 6-$CH_3$ | H | Cl | 5-$CF_3$-pyridyl -2 | $CH_2C{\equiv}CH$ |
| 652 | 6-$CH_3$ | H | Cl | 2-Pyrazinyl | $CH_2C{\equiv}CH$ |
| 653 | 6-$CH_3$ | $CH_3$ | H | Cyclohexyl | $CH_2C{\equiv}CH$ |
| 654 | 6-$CH_3$ | $CH_3$ | H | Benzyl | $CH_2C{\equiv}CH$ |
| 655 | 6-$CH_3$ | $CH_3$ | H | Phenyl | $CH_2C{\equiv}CH$ |
| 656 | 6-$CH_3$ | $CH_3$ | H | 2-Pyridyl | $CH_2C{\equiv}CH$ |
| 657 | 6-$CH_3$ | $CH_3$ | H | 5-Cl-pyridyl-2 | $CH_2C{\equiv}CH$ |
| 658 | 6-$CH_3$ | $CH_3$ | H | 5-$CF_3$-pyridyl -2 | $CH_2C{\equiv}CH$ |
| 659 | 6-$CH_3$ | $CH_3$ | H | 2-Pyrazinyl | $CH_2C{\equiv}CH$ |
| 660 | 3-F | $CH_3$ | H | Cyclohexyl | $C_2H_5$ |
| 661 | 3-F | $CH_3$ | H | Benzyl | $C_2H_5$ |
| 662 | 3-F | $CH_3$ | H | Phenyl | $C_2H_5$ |
| 663 | 3-F | $CH_3$ | H | 2-Pyridyl | $C_2H_5$ |
| 664 | 3-F | $CH_3$ | H | 5-Cl-pyridyl-2 | $C_2H_5$ |
| 665 | 3-F | $CH_3$ | H | 5-$CF_3$-pyridyl -2 | $C_2H_5$ |
| 666 | 3-F | $CH_3$ | H | 2-Pyrazinyl | $C_2H_5$ |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:

aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa Gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;

aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;

aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;

aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;

aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;

aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor:

aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;

aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;

aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;

aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;

aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Verbindungen I können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:

* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,

* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz z.B. zum Schutz von Holz, Papier und Textilien eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:

- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;

- Trägerstoffe wie natürliche Gesteinsmehle (z.B. Kaoline, Tonerden, Talkum, Kreide) und synthetische Gesteinsmehle (z.B. hochdisperse Kieselsäure, Silikate);

- Emulgiermittel wie nichtionogene und anionische Emulgatoren (z.B. Polyoxyethylen-Fettalkohol-Ether, Alkylsulfonate und Arylsulfonate) und

- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;

II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.

III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.

IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.

V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;

VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;

VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;

X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsoure-di-isopropylester;

heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylamino-benzimidezol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-l-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,

sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetylD,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-l,2,4-triazol.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

1. N-(2-(N'-(p-Methylphenyl)-4'-chlor-pyrazolyl-3'-oxymethyl)-phenyl)-N-methoxy-carbaminsäuremethylester (Tabelle, Nr. 19)

Eine Mischung von 1,7 g (Reinheit ca. 75 %ig, ≙ 4,6 mmol) N-(2-Brommethylphenyl)-N-methoxy-carbaminsäuremethylester (WO 93/15046), 1 g (4,8 mmol) N-(p-Methylphenyl)-4-chlor-3-hydroxypyrazol und 1 g (7,2 mmol) $K_2CO_3$ in 15 ml Dimethylformamid wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden mit Wasser extrahiert, über $MgSO_4$ getrocknet und eingeengt. Dann wird der Rückstand mit Methylenchlorid über $Al_2O_3$ und anschließend mit Cyclohexan/Essigester-Gemischen über Kieselgel chromatographiert. Man erhält 1,4 g (68 %) der Titelverbindung als hellgelbes Öl.

$^1$H-NMR($CDCl_3$; δ in ppm): 7,75 (s, 1H, Pyrazolyl); 7,70 (m, 1H, Phenyl); 7,5 (m, 5H, Phenyl); 7,2 (d, 2H, Phenyl); 5,4 (s, 2H, $OCH_2$); 3,75, 3,8 (2s, je 3H, 2 x $OCH_3$); 2,35 (s, 3H, $CH_3$)

2. N-Methyl-N"-methoxy-N"-(2-((N"-pyrazinyl)-pyrazolyl-3"-oxymethyl)-phenyl)-harnstoff (Tabelle, Nr. 32)

a) N-Hydroxy-N-(2-methylphenyl)-carbaminsäurephenylester

Eine Mischung von 350 g (Reinheit ca. 80%ig; 2,3 mol; hergestellt analog Bamberger et al., Ann. Chem. <u>316</u> (1901), 278) N-(2-Methylphenyl)-hydroxylamin und 286,8 g (3,4 mol) $NaHCO_3$ in 700 ml $CH_2Cl_2$ wird bei ca. -10°C unter kräftigem Rühren mit 447 g (2,85 mol) Phenylchlorformiat versetzt. Man rührt ca. eine Stunde bei -10°C und tropft anschließend 600 ml Wasser hinzu, wobei sich die Temperatur der Reaktionsmischung auf 5-10°C erhöht und eine starke Gasentwicklung eintritt. Dann wird die wäßrige Phase abgetrennt und noch einmal mit $CH_2Cl_2$ extrahiert. Die vereinigten organischen Phasen werden mit Wasser extrahiert, über $MgSO_4$ getrocknet und eingeengt. Der Rückstand kristallisiert und wird mit Cyclohexan ausgerührt. Man erhält 407 g (72 %) der Titelverbindung als farblosen Festkörper.

$^1$H-NMR($CDCl_3$; δ in ppm): 8,6 (s, breit, 1H, OH); 7,0-7,4 (m, 9H, Phenyl), 2,4(s,3H,$CH_3$)

b) N-Methoxy-N-(2-methylphenyl)-carbaminsäurephenylester

Eine Mischung von 407 g (1,6 mol) N-Hydroxy-N-(2-methylphenyl)-carbaminsäurephenylester (Beispiel 2a) und 277 g (2,0 mol) $K_2CO_3$ in 700 ml $CH_2Cl_2$ wird tropfenweise mit 211 g (1,67 mol) Dimethylsulfat versetzt. Dabei erwärmt sich die Reaktionsmischung auf ca. 40°C. Man rührt über Nacht bei Raumtemperatur und filtriert anschließend die Reaktionsmischung über Kieselgur. Das Filtrat wird mit $NH_3$-Lsg. und Wasser gewaschen, über $MgSO_4$ getrocknet und eingeengt. Der Rückstand kristallisiert und wird mit Hexan ausgerührt. Man erhält 324 g (75 %) der Titelverbindung als farblosen Festkörper.

$^1$H-NMR($CDCl_3$ ; δ in ppm): 7,1 - 7,6 (m, 9H, Phenyl); 3,8 (s,3H,$OCH_3$); 2,4 (s, 3H, $CH_3$)

c) N-Methoxy-N-(2-brommethylphenyl)-carbaminsäurephenylester

Eine Mischung von 324 g (1,3 mol) N-Methoxy-N-(2-methylphenyl)-carbaminsäurephenylester (Beispiel 2b), 258 g (1,45 mol) N-Bromsuccinimid und 1 g Azoisobutyrodinitril in 1 l $CCl_4$ wird ca. 6 Stunden mit einer 300 W UV-Lampe bestrahlt, wodurch die Reaktionsmischung zum Sieden erhitzt wird. Anschließend gibt man 13 g N-Bromsuccinimid hinzu und bestrahlt weitere 8 Stunden. Dann kühlt man auf Raumtemperatur und filtriert das ausgefallene Succinimid ab. Anschließend wird die organische Phase mit Wasser extrahiert, über $MgSO_4$ getrocknet und eingeengt. Der Rückstand kristallisiert und wird mit Cyclohexan ausgerührt. Man erhält 300 g (68 %) der Tielverbindung als beigen Festkörper.

$^1$H-NMR($CDCl_3$ ; δ in ppm): 7,0 - 7,6 (m, 9H, Phenyl);4,65 (s,2H,$CH_2$-Br); 3,9 (s, 3H, $OCH_3$)

d) N-Methoxy-N- (2-((N'-pyrazinyl)-pyrazolyl-3'-oxymethyl)-phenyl)-carbaminsäurephenylester

Eine Mischung von 3,1 g (9,2 mmol) N-Methoxy-N-(2-brommethylphenyl)-carbaminsäurephenylester (Beispiel 2c), 1,5 g (9,2 mmol) N-Pyrazinyl-3-hydroxypyrazol und 2 g (14,5 mmol) $K_2CO_3$ in 10 ml DMF wird über Nacht bei Raumtemperatur gerührt. Anschließend wird die Reaktionsmischung mit Wasser verdünnt und dreimal mit Methyl-t-butylether extrahiert. Die vereinigten organischen Phasen werden mit Wasser extrahiert, über $MgSO_4$ getrocknet und eingeengt. Der Rückstand wird säulenchromatographisch mit Cyclohexan/Essigester-Gemischen gereinigt. Man erhält 2,4 g (63 %) der Titelverbindung als gelbes Öl.

$^1$H-NMR($CDCl_3$; δ in ppm): 9,15 (d, 1H, Pyrazolyl); 8,3 (m, 3H, Pyrazinyl); 7,7 (m, 1H, Phenyl); 7,1-7,6

(m, 8H, Phenyl); 6,0 (d, 1H, Pyrazolyl); 5,5 (s, 2H, $OCH_2$); 3,85 (s, 3H, $OCH_3$)

e) N-Methyl-N"-methoxy-N"-(2-((N"-pyrazinyl)-pyrazolyl-3"-oxymethyl)-phenyl)-harnstoff

Eine Mischung von 1,9 g (4,6 mmol) N-Methoxy-N-(2-((N'-pyrazinyl)-pyrazolyl-3'-oxymethyl)-phenyl)-carbaminsäure-phenylester (Beispiel 2d) und 15 ml wäßriger Methylamin-Lösung (40 %ig) wird über Nacht bei Raumtemperatur gerührt. Anschließend gibt man Wasser hinzu und extrahiert die wäßrige Phase zweimal mit Methylenchlorid. Die vereinigten organischen Phasen werden mit Wasser gewaschen, über $MgSO_4$ getrocknet und eingeengt. Der Rückstand kristallisiert und wird mit Cyclohexan ausgerührt. Man erhält 0,9 g (55 %) der Titelverbindung als beigen Festkörper.

$^1$H-NMR($CDCl_3$; $\delta$ in in ppm): 9,15 (d, 1H, Pyrazolyl); 8,3 (m, 3H, Pyrazinyl); 7,6 (m, 1H, Phenyl); 7,35 (m, 3H, Phenyl); 6,0 (m, 2H, NH, Pyrazolyl); 5,45 (s, 2H, $OCH_2$); 3,7 (s, 3H, $OCH_3$); 2,9 (d, 3H, $NCH_3$)

Tabelle:

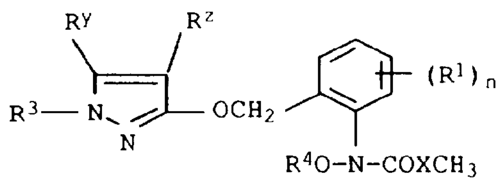

I.A

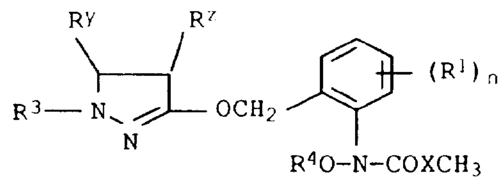

I.B

| Nr. | Struktur | $R^1_n$ | $R^Y$ | $R^Z$ | $R^3$ | $R^4$ | X | Fp [°C], IR [$cm^{-1}$] |
|---|---|---|---|---|---|---|---|---|
| 1 | I.A | H | H | H | $C_6H_5$ | $CH_3$ | O | 1737, 1600, 1493, 1480, 1456, 1358, 1332, 755 |
| 2 | I.A | H | H | H | 4-Cl-$C_6H_4$ | $CH_3$ | O | 1737, 1547, 1503, 1480, 1457, 1441, 1350, 1094, 1030, 936 |
| 3 | I.A | H | H | H | 2,4-$Cl_2$-$C_6H_3$ | $CH_3$ | O | 1739, 1547, 1492, 1475, 1457, 1440, 1356, 1107, 1058, 1027 |
| 4 | I.A | H | H | H | 2-$CH_3$-$C_6H_4$ | $CH_3$ | O | 1739, 1710, 1542, 1482, 1457, 1440, 1358, 1052, 1030, 763 |
| 5 | I.A | H | H | H | 3-$CH_3$-$C_6H_4$ | $CH_3$ | O | 1738, 1593, 1545, 1494, 1483, 1457, 1441, 1357, 1056, 1032 |
| 6 | I.A | H | H | H | 4-$CH_3$-$C_6H_4$ | $CH_3$ | O | 1738, 1544, 1519, 1482, 1456, 1440, 1393, 1358, 1243, 1031, |

| Nr. | Struktur | $R^1_n$ | $R^Y$ | $R^Z$ | $R^3$ | $R^4$ | X | Fp [°C], IR [$cm^{-1}$] |
|---|---|---|---|---|---|---|---|---|
| 7 | I.A | H | H | H | 2-Cl-$C_6H_4$ | $CH_3$ | O | 1739, 1710, 1546, 1495, 1476, 1453, 1441, 1358, 1027, 757 |
| 8 | I.A | H | H | H | 3-Cl-$C_6H_4$ | $CH_3$ | O | 1736, 1597, 1548, 1495, 1476, 1456, 1440, 1357, 1101, 771 |
| 9 | I.A | H | H | H | 2,6-$Cl_2$-$C_6H_3$ | $CH_3$ | O | 1727, 1543, 1464, 1445, 1364, 1348, 791, 785, 749, |
| 10 | I.A | H | H | H | 3,5-$Cl_2$-$C_6H_4$ | $CH_3$ | O | 120 |
| 11 | I.A | H | H | H | 2,5-$Cl_2$-$C_6H_3$ | | O | 1737, 1710, 1547, 1489, 1471, 1456, 1437, 1346, 1096, 1027 |
| 12 | I.A | H | H | H | 3,4-$Cl_2$-$C_6H_3$ | $CH_3$ | O | 85 |
| 13 | I.A | H | H | H | 2-$CH_3$,4-Cl-$C_6H_3$ | $CH_3$ | O | 1738, 1710, 1543, 1494, 1480, 1457, 1441, 1358, 1100, 940 |
| 14 | I.A | H | H | H | 3-$CF_3$-$C_6H_4$ | $CH_3$ | O | 1721, 1558, 1459, 1441, 1368, 1333, 1121, 1067, 793, 764 |
| 15 | I.A | H | H | H | 4-$OCH_3$-$C_6H_4$ | $CH_3$ | O | 1737, 1541, 1517, 1483, 1457, 1442, 1359, 1250, 1056, 1032 |
| 16 | I.A | H | $CH_3$ | H | $C_6H_5$ | $CH_3$ | O | 1739, 1710, 1560, 1504, 1484, 1456, 1440, 1380, 1359, 760 |
| 17 | I.A | H | H | $CH_3O$-CO | $C_6H_5$ | $CH_3$ | O | 1720, 1702, 1570, 1540, 1446, 1372, 1357, 1285, 1119, 751 |
| 18 | I.A | H | H | H | 4-F-$C_6H_4$ | $CH_3$ | O | 1737, 1546, 1516, 1482, 1457, 1440, 1359, 1233, 1031, 835 |

| Nr. | Struktur | $R^1_n$ | $R^Y$ | $R^Z$ | $R^3$ | $R^4$ | X | Fp [°C], IR [$cm^{-1}$] |
|---|---|---|---|---|---|---|---|---|
| 19 | I.A | H | H | Cl | 4-$CH_3$-$C_6H_4$ | $CH_3$ | O | 1738, 1554, 1509, 1456, 1440, 1358, 1253, 1118, 940, 760 |
| 20 | I.A | H | 3-F | H | 2,4-$Cl_2$-$C_6H_3$ | $CH_3$ | O | 90 |
| 21 | I.A | H | 3-F | H | 2,4-$Cl_2$-$C_6H_3$ | $CH_3$ | O | 109 |
| 22 | I.A | H | H | H | 3-$OCH_3$-$C_6H_4$ | $CH_3$ | O | 1737, 1607, 1597, 1545, 1499, 1482, 1472, 1440, 1358 |
| 23 | I.A | H | H | Cl | 2,4-$Cl_2$-$C_6H_3$ | $CH_3$ | O | 110 |
| 24 | I.A | H | $CF_3$ | H | 2,4-$Cl_2$-$C_6H_4$ | $CH_3$ | O | 1739, 1507, 1486, 1457, 1359, 1250, 1190, 1139, 1109, 1092 |
| 25 | I.A | H | $CH_3$ | H | 2,4-$Cl_2$-$C_6H_3$ | $CH_3$ | O | 1738, 1710, 1567, 1561, 1500, 1484, 1456, 1440, 1359, 1104 |
| 26 | I.A | H | H | Cl | 4-Cl-$C_6H_4$ | $CH_3$ | O | 1729, 1553, 1511, 1497, 1438, 1356, 1332, 1265, 1122, 1112 |
| 27 | I.A | H | H | H | 3,4-($OCF_2O$)-$C_6H_3$ | $CH_3$ | O | 97 |
| 28 | I.A | H | H | H | 2-pyridyl | $CH_3$ | O | 85 |
| 29 | I.A | H | H | H | 5-$CF_3$-pyridyl-2 | $CH_3$ | O | 81 |
| 30 | I.A | H | H | H | 2-pyrazinyl | $CH_3$ | O | 87 |
| 31 | I.B | H | H | H | $C_6H_5$ | $CH_3$ | O | 1739, 1639, 1599, 1501, 1456, 1439, 1411, 1354, 1252, 752 |
| 32 | I.A | H | H | H | 2-pyrazinyl | $CH_3$ | NH | 145 |
| 33 | I.A | H | H | $NO_2$ | 5-$CF_3$-pyri-dyl-2 | $CH_3$ | O | 126 |

| Nr. | Struktur | $R^1_n$ | $R^y$ | $R^z$ | $R^5$ | $R^4$ | X | Fp [°C], IR [cm$^{-1}$] |
|---|---|---|---|---|---|---|---|---|
| 34 | I.A | H | $CH_3$ | H | 4-Cl-$C_6H_4$ | $CH_3$ | O | 1738, 1561, 1500, 1456, 1440, 1359, 1094, 1010, 764 |
| 35 | I.A | H | H | H | 6-Cl-Pyridazin-3-yl | $CH_3$ | O | 135 |
| 36 | I.A | H | H | H | 5-Cl-Pyridin-2-yl | $CH_3$ | O | 77 |
| 37 | I.A | H | H | $CF_3$ | Cyclohexyl | $CH_3$ | O | 1743, 1496, 1456, 1441, 1359, 1272, 1262, 1176, 1124, 1029 |
| 38 | I.A | H | H | Cl | 5-Cl-Pyridin-2-yl | $CH_3$ | O | 92 |
| 39 | I.A | H | $H_3CO_2C$ | H | $CH_2C_6H_5$ | $CH_3$ | O | 71 |
| 40 | I.A | H | H | H | $C_6H_5$ | H | O | 1718, 1600, 1545, 1507, 1481, 1458, 1399, 1359, 1032, 757 |
| 41 | I.A | H | H | H | $C_6H_5$ | $CH_3$ | NH | 1675, 1600, 1545, 1508, 1479, 1462, 1399, 1355, 1054, 756 |
| 42 | I.A | H | H | H | $C_6H_5$ | $CH_3$ | – | 1680, 1600, 1545, 1507, 1480, 1456, 1359, 1056, 1032, 758 |
| 43 | I.A | H | H | H | $C_6H_5$ | H | NH | 1653, 1601, 1545, 1707, 1479, 1454, 1414, 1398, 1355, 755 |
| 44 | I.A | H | H | H | $C_6H_5$ | $CH_3$ | $CH_2$ | 1678, 1600, 1545, 1480, 1456, 1394, 1378, 1358, 1055, 756 |
| 45 | I.A | H | H | H | $C_6H_5$ | H | – | 1643, 1622, 1601, 1544, 1493, 1480, 1368, 1027, 759, 745 |
| 46 | I.A | H | H | H | $C_6H_5$ | H | $CH_2$ | 1619, 1600, 1550, 1495, 1482, 1462, 1454, 1358, 765, 753 |
| 47 | I.A | H | H | H | 2,4-$Cl_2$-Phenyl | $CH_3$ | NH | 130 |

| Nr. | Struktur | $R^1_n$ | $R^y$ | $R^z$ | $R^3$ | $R^4$ | X | Fp [°C], IR [$cm^{-1}$] |
|---|---|---|---|---|---|---|---|---|
| 48 | I.A | H | H | H | 4-Cl-$C_6H_4$ | H | O | 105 |
| 49 | I.A | H | H | H | 4-Cl-$C_6H_4$ | H | NH | 1653, 1546, 1503, 1480, 1455, 1426, 1390, 1357, 1094, 1071 |
| 50 | I.A | H | H | H | 4-Cl-$C_6H_4$ | $CH_3$ | NH | 1675, 1546, 1503, 1479, 1457, 1425, 1389, 1355, 936, 829 |
| 51 | I.A | H | H | H | $CH_2C_6H_5$ | $CH_3$ | O | 1737, 1709, 1537, 1488, 1456, 1439, 1359, 1325, 1032, 733 |
| 52 | I.A | H | H | H | $CH_2$-[4-Cl-$C_6H_4$] | $CH_3$ | O | 1735, 1709, 1538, 1492, 1456, 1439, 1358, 1323, 1096, 761 |
| 53 | I.A | H | H | H | 2,4-$(CH_3)_2$-$C_6H_3$ | $CH_3$ | O | 92 |
| 54 | I.A | H | H | Cl | 2-Pyrazinyl | $CH_3$ | O | 114 |
| 55 | I.B | H | H | H | 4-Cl-$C_6H_4$ | $CH_3$ | O | 1740, 1639, 1599, 1495, 1456, 1439, 1415, 1355, 1251, 1092 |
| 56 | I.A | H | H | H | 2-Cl, 4-F-$C_6H_3$ | $CH_3$ | O | 1737, 1547, 1505, 1494, 1480, 1457, 1441, 1358, 1258, 1028 |

Beispiele zur Wirkung gegen Schadpilze

Die fungizide Wirkung der Verbindungen der Formel I ließ sich durch folgende Versuche zeigen:
Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Wirksamkeit gegen Puccinia recondita

Blätter von Weizensämlingen (Sorte "Kanzler") wurden mit Sporen des Braunrosts (Puccinia recondita) bestäubt. Die so behandelten Pflanzen wurden 24 h bei 20-22°C und einer relativen Luftfeuchtigkeit von 90-95% inkubiert und anschließend mit der wäßrigen Wirkstoffaufbereitung (63 ppm Wirkstoff) behandelt. Nach weiteren 8 Tagen bei 20-22°C und 65-70% relativer Luftfeuchtigkeit wurde das Ausmaß der Pilzentwicklung ermittelt. Die Auswertung erfolgte visuell.
In diesem Test zeigten die mit den erfindungsgemäßen Verbindungen 2-6, 8, 11-15, 18-20, 22, 23 und 26-29 behandelten Pflanzen einen Befall von 5% und weniger, während die mit einer aus WO-A 93/15,046 bekannten Verbindung (Tabelle 7, Beispiel Nr. 8) behandelten Pflanzen zu 25% befallen waren. Die unbehandelten Pflanzen waren zu 70% befallen.
In einem entsprechenden Versuch zeigten die mit 250 ppm der erfindungsgemäßen Verbindung Nr. 1 behandelten Pflanzen einen Befall von 3% während Pflanzen, die mit 250 ppm einer aus WO-A 93/15,046 bekannten Verbindung (Tabelle 7, Nr. 21) behandelt waren ebenso wie die unbehandelten Pflanzen zu 70% befallen waren.
In einem entsprechenden Versuch zeigten die mit 250 ppm der erfindungsgemäßen Verbindungen Nr. 1-8, 10-16, 18-20, 22, 23, 27-30, 34, 36-38, 41, 47 und 51-56 behandelten Pflanzen einen Befall von 15% und weniger während Pflanzen, die mit 250 ppm einer aus WO-A 93/15,046 bekannten Verbindung (Tabelle 7, Nr. 21) behandelt waren ebenso wie die unbehandelten Pflanzen zu 70% befallen waren.

Wirksamkeit gegen Plasmopara viticola

Topfreben (Sorte: "Müller Thurgau") wurden mit der Wirkstoffaufbereitung tropfnaß gespritzt. Nach 8 Tagen wurden die Pflanzen mit einer Zoosporenaufschwemmung des Pilzes *Plasmopara viticola* besprüht und 5 Tage bei 20-30 °C bei hoher Luftfeuchtigkeit bewahrt. Vor der Beurteilung wurden die Pflanzen danach für 16h bei hoher Luftfeuchtigkeit bewahrt.
Die Auswertung erfolgte visuell.
In diesem Test zeigten die mit den erfindungsgemäßen Verbindungen 1-3, 5, 6, 13, 15 und 29 behandelten Pflanzen einen Befall von 10% und weniger, während die mit einer aus WO-A 39/15,046 bekannten Verbindung (Tabelle 7, Beispiel Nr. 8) behandelten Pflanzen zu 25% befallen waren. Die unbehandelten Pflanzen waren zu 70% befallen.

Wirksamkeit gegen Botrytis cinerea (Grauschimmel)

Paprikasämlinge (Sorte: "Neusiedler Ideal Elite") mit 4-5 Blättern wurden mit der Wirkstoffaufbereitung (Aufwandmenge: 500 ppm) tropfnaß gespritzt. Nach dem Abtrocknen wurden die Pflanzen mit einer Konidienaufschwemmung des Pilzes *Botrytis cinerea* besprüht und 5 Tage bei 22-24 °C bei hoher Luftfeuchtigkeit bewahrt. Die Auswertung erfolgte visuell.
In diesem Test zeigten die mit der erfindungsgemäßen Verbindung 1 behandelten Pflanzen keinen Befall, während die mit einer aus WO-A 93/15,046 bekannten Verbindung (Tabelle 7, Beispiel Nr. 21) behandelten Pflanzen zu 70% befallen waren. Die unbehandelten Pflanzen waren zu 80% befallen.

Wirksamkeit gegen Erysiphe graminis var. tritici

Blätter von Weizenkeimlingen (Sorte "Frühgold") wurden zunächst mit der wäßrigen Aufbereitung (Aufwandmenge 250 ppm) der Wirkstoffe behandelt. Nach ca. 24 h wurden die Pflanzen mit Sporen des Weizenmehltaus *(Erysiphe graminis var. tritici)* bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittlelt.
In diesem Test zeigten die mit der erfindungsgemäßen Verbindung Nr. 1 behandelten Pflanzen keinen Befall, während die mit einer aus WO-A 93/15,046 bekannten Verbindung (Tabelle 7, Nr. 21) behandelten Pflanzen zu 25% befallen waren. Die unbehandelten Pflanzen waren zu 70% befallen.
In einem entsprechenden Versuch zeigten die mit 250 ppm der erfindungsgemäßen Verbindungen Nr. 1-7, 10, 13, 14, 18-20, 27-29, 34, 36, 41, 50 und 56 behandelten Pflanzen einen Befall von 15% und weniger während Pflanzen,

die mit 250 ppm einer aus WO-A 93/15,046 bekannten Verbindung (Tabelle 7, Nr. 21) behandelt waren zu 25% befallen waren. Die unbehandelten Pflanzen waren zu 70% befallen.

In einem entsprechenden Versuch zeigten die mit 63 ppm der erfindungsgemäßen Verbindungen Nr. 1-7, 10, 13, 14, 18-20, 27-29, 34, 36, 41, 50 und 56 behandelten Pflanzen einen Befall von 15% und weniger während Pflanzen, die mit 250 ppm einer aus WO-A 93/15,046 bekannten Verbindung (Tabelle 7, Nr. 21) behandelt waren zu 40% befallen waren. Die unbehandelten Pflanzen waren zu 70% befallen.

In einem entsprechenden Versuch zeigten die mit 16 ppm der erfindungsgemäßen Verbindungen Nr. 1-7, 10, 13, 14, 18-20, 27-29, 34, 36, 41, 50 und 56 behandelten Pflanzen einen Befall von 25% und weniger während Pflanzen, die mit 250 ppm einer aus WO-A 93/15,046 bekannten Verbindung (Tabelle 7, Nr. 21) behandelt waren zu 65% befallen waren. Die unbehandelten Pflanzen waren zu 70% befallen.

Beispiele zur Wirkung gegen tierische Schädlinge

Die Wirkung der Verbindungen der allgemeinen Formel I gegen tierische Schädlinge ließ sich durch folgende Versuche zeigen:
Die Wirkstoffe wurden

a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

**1.** 2-[(Dihydro)pyrazolyl-3'-oxymethylen]-anilide der Formel I

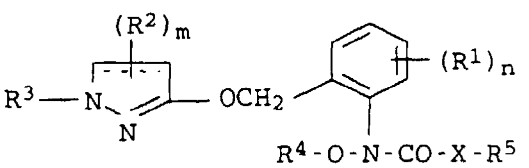

I

in der -- für eine Einfach- oder Doppelbindung steht und die Indices und die Substituenten die folgende Bedeutung haben:

n 0, 1, 2, 3 oder 4, wobei die Substituenten $R^1$ verschieden sein können, wenn n größer als 1 ist;

m 0, 1 oder 2, wobei die Substituenten $R^2$ verschieden sein können, wenn m größer als 1 ist;

X eine direkte Bindung, O oder $NR^a$;

$R^a$ Wasserstoff, Alkyl, Alkenyl, Alkinyl, Cycloalkyl oder Cycloalkenyl;

$R^1$ Nitro, Cyano, Halogen,

ggf. subst. Alkyl, Alkenyl, Alkinyl, Alkoxy, Alkenyloxy, Alkinyloxy oder

für den Fall, daß n für 2 steht zusätzlich eine an zwei benachbarte Ringatome gebundene ggf. subst. Brücke, welche drei bis vier Glieder aus der Gruppe 3 oder 4 Kohenstoffatome, 1 bis 3 Kohlenstoffatome

und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann;

$R^2$ Nitro, Cyano, Halogen, $C_1$-$C_4$-Alkyl, $C_1$-$C_4$-Halogenalkyl, $C_1$-$C_4$-Alkoxy, $C_1$-$C_4$-Alkylthio oder $C_1$-$C_4$-Alkoxycarbonyl;

$R^3$ ggf. subst. Alkyl, Alkenyl oder Alkinyl;

ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, oder
ein ggf. subst. ein- oder zweikerniger aromatischer Rest, welcher neben Kohlenstoffatomen ein bis vier Stickstoffatome oder ein oder zwei Stickstoffatome und ein Sauerstoff- oder Schwefelatom oder ein Sauerstoff- oder
Schwefelatom als Ringglieder enthalten kann;

$R^4$ Wasserstoff,
ggf. subst. Alkyl, Alkenyl, Alkinyl, Cycloalkyl, Cycloalkenyl, Alkylcarbonyl oder Alkoxycarbonyl;

$R^5$ Alkyl, Alkenyl, Alkinyl, Cycloalkyl oder Cycloalkenyl, oder
für den Fall, daß X für $NR^a$ steht, zusätzlich Wasserstoff.

**2.** Verfahren zur Herstellung der Verbindungen der Formel I gemäß Anspruch 1, in denen $R^4$ Wasserstoff bedeutet, und X für eine direkte Bindung oder Sauerstoff steht, dadurch gekennzeichnet, daß man ein Benzylderivat der Formel II,

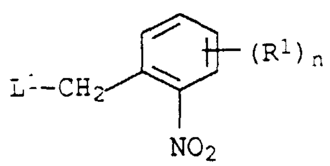

II

in der $L^1$ eine nucleophil austauschbare Gruppe bedeutet in Gegenwart einer Base mit einem 3-Hydroxy(dihydro)pyrazol der Formel III

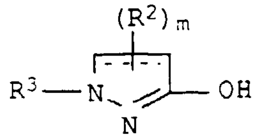

III

in das entsprechende 2-[(Dihydro)pyrazolyl-3'-oxymethylen]-nitrobenzol der Formel IV

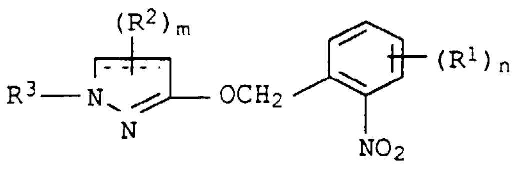

IV

überführt, IV anschließend zum N-Hydroxylanilin der Formel Va

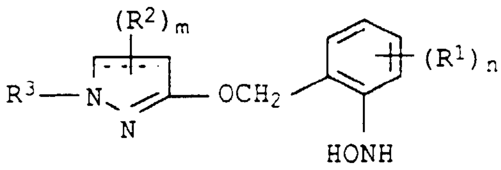

Va

reduziert und Va mit einer Carbonylverbindung der Formel VI

$$L^2\text{-CO-X-R}^5 \qquad VI$$

in der $L^2$ Halogen bedeutet und X für eine direkte Bindung oder Sauerstoff steht, in I umwandelt.

**3.** Verfahren zur Herstellung der Verbindungen der Formel I gemäß Anspruch 1, in denen $R^4$ nicht Wasserstoff bedeutet und X für eine direkte Bindung oder Sauerstoff steht, dadurch gekennzeichnet, daß man ein Benzylderivat der Formel IIa

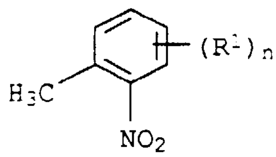

IIa

zunächst zum entsprechenden Hydroxyanilin der Formel Vb

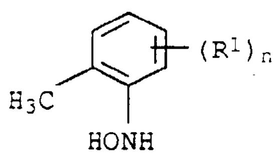

Vb

reduziert, Vb mit einer Carbonylverbindung der Formel VI gemäß Anspruch 2 in das entsprechende Anilid der Formel VII

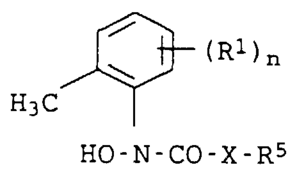

VII

überführt, VII anschließend mit einer Verbindung VIII

$$L^3\text{-R}^4 \qquad VIII$$

in der $L^3$ eine nucleophil austauschbare Gruppe bedeutet und $R^4$ nicht für Wasserstoff steht, in das Amid der Formel IX

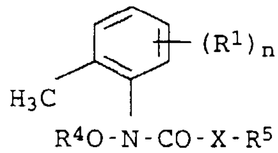

IX

überführt, IX anschließend in das entsprechende Benzylhalogenid der Formel X

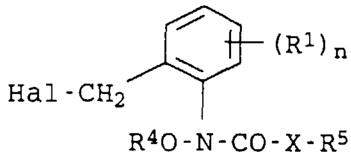

X

in der Hal für ein Halogenatom steht, überführt und X in Gegenwart einer Base mit einem 3-Hydroxy(dihydro)pyrazol der Formel III gemäß Anspruch 2 in I umwandelt.

**4.** Verfahren zur Herstellung der Verbindungen I gemäß Anspruch 1, in denen $R^4$ nicht Wasserstoff bedeutet und X für eine direkte Bindung oder Sauerstoff steht, dadurch gekennzeichnet, daß man eine entsprechende Verbindung der Formel I, in der $R^4$ Wasserstof bedeutet, mit einer Verbindung der Formel VIII gemäß Anspruch 3 umsetzt.

**5.** Verfahren zur Herstellung der Verbindungen der Formel I gemäß Anspruch 1, in denen X für $NR^a$ steht, dadurch gekennzeichnet, daß man ein Benzylanilid der Formel IXa

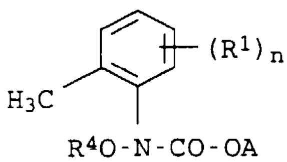

IXa

in der A für Alkyl oder Phenyl steht, in das entsprechende Benzylhalogenid der Formel Xa

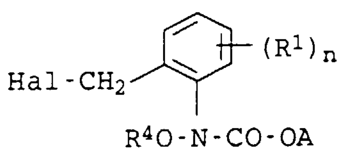

Xa

in der Hal für ein Halogenatom steht, überführt, Xa in Gegenwart einer Base mit einem 3-Hydroxy(dihydro)pyrazol der Formel III gemäß Anspruch 2 in eine Verbindung der Formel I.A

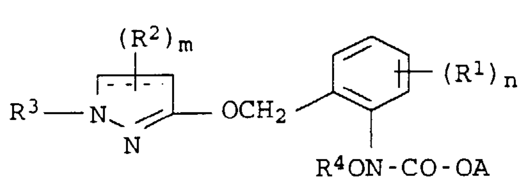

I.A

überführt und I.A anschließend mit einem Amin der Formel XI

$$H_2NR^a \quad (XIa)$$

$$HNR^aR^5 \quad (XIb)$$

zu I umsetzt.

**6.** Zwischenprodukte der Formel XIIa

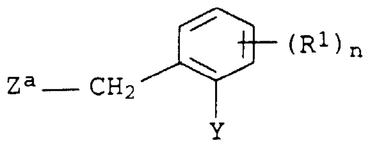

XIIa

in der die Substituenten und der Index die folgende Bedeutung haben:

n 0, 1, 2, 3 oder 4, wobei die Substituenten $R^1$ verschieden sein können, wenn n größer als 1 ist;

$R^1$ Nitro, Cyano, Halogen,

ggf. subst. Alkyl, Alkenyl, Alkinyl, Alkoxy, Alkenyloxy, Alkinyloxy oder

für den Fall, daß n für 2 steht zusätzlich eine an zwei benachbarte Ringatome gebundene ggf. subst. Brücke, welche drei bis vier Glieder aus der Gruppe 3 oder 4 Kohlenstoffatome, 1 bis 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann;

Y $NO_2$, NHOH oder $NHOR^4$

$R^4$ ggf. subst. Alkyl, Alkenyl, Alkinyl, Cycloalkyl, Cycloalkenyl, Alkylcarbonyl oder Alkoxycarbonyl;

$Z^a$ eine Gruppe

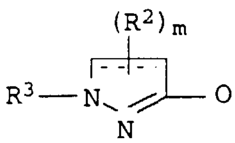

$Z^a$

m 0, 1 oder 2, wobei die Substituenten $R^2$ verschieden sein können, wenn m größer als 1 ist;

$R^2$ Nitro, Cyano, Halogen, $C_1$-$C_4$-Alkyl, $C_1$-$C_4$-Halogenalkyl, $C_1$-$C_4$-Alkoxy, $C_1$-$C_4$-Alkylthio oder $C_1$-$C_4$-Alkoxycarbonyl;

$R^3$ ggf. subst. Alkyl, Alkenyl oder Alkinyl;

ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, oder

ein ggf. subst. ein- oder zweikerniger aromatischer Rest, welcher neben Kohlenstoffatomen ein bis vier Stickstoffatome oder ein oder zwei Stickstoffatome und ein Sauerstoff- oder Schwefelatom

oder ein Sauerstoff- oder Schwefelatom als Ringglieder enthalten kann.

**7.** Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

**8.** Zwischenprodukte der allgemeinen Formel XIII

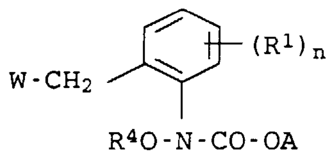

XIII

wobei die Substituenten $R^1$ und $R^4$ sowie der Index n die in Anspruch 1 gegebene Bedeutung haben und die Substituenten W und A die folgende Bedeutung haben:

W eine Gruppe $Z^a$, welche die in Anspruch 6 gegebene Bedeutung hat, und
A Phenyl.

**9.** Verwendung der Verbindungen I gemäß Anspruch 1 zur Herstellung eines zur Bekämpfung von tierischen Schädlingen oder Schadpilzen geeigneten Mittels.

**10.** Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

**11.** Verfahren zur Bekämpfung von tierischen Schädlingen, dadurch gekennzeichnet, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

**1.** A 2-[(dihydro)pyrazol-3'-yloxymethylene]anilide of the formula I

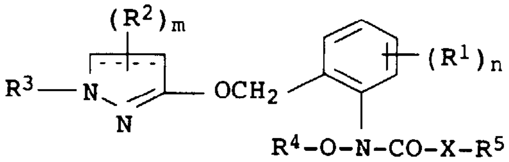

I

where -- is a single or double bond and the indices and the substituents have the following meanings:

n is 0, 1, 2, 3 or 4, it being possible for the substituents $R^1$ to be different if n is greater than 1;

m is 0, 1 or 2, it being possible for the substituents $R^2$ to be different if m is greater than 1;

X is a direct bond, O or $NR^a$;

$R^a$ is hydrogen, alkyl, alkenyl, alkynyl, cycloalkyl or cycloalkenyl;

$R^1$ is nitro, cyano, halogen,

unsubstituted or substituted alkyl, alkenyl, alkynyl, alkoxy, alkenyloxy, alkynyloxy or

in the case where n is 2, additionally is an unsubstituted or substituted bridge bonded to two adjacent ring atoms and containing three to four members from the group consisting of 3 or 4 carbon atoms, 1 to 3 carbon atoms and 1 or 2 nitrogen, oxygen and/or sulfur atoms, this bridge together with the ring to which it is bonded being able to form a partly unsaturated or aromatic radical;

$R^2$ is nitro, cyano, halogen, $C_1$-$C_4$-alkyl, $C_1$-$C_4$-haloalkyl, $C_1$-$C_4$-alkoxy, $C_1$-$C_4$-alkylthio or $C_1$-$C_4$-alkoxycarbonyl;

$R^3$ is unsubstituted or substituted alkyl, alkenyl or alkynyl;

an unsubstituted or substituted, saturated or mono- or diunsaturated ring which, in addition to carbon atoms, can contain one to three of the following heteroatoms as ring members: oxygen, sulfur and nitrogen, or
an unsubstituted or substituted, mono- or binuclear aromatic radical which, in addition to carbon atoms, can contain one to four nitrogen atoms or one or two nitrogen atoms and one oxygen or sulfur atom or one oxygen or sulfur atom as ring members;

$R^4$ is hydrogen,
unsubstituted or substituted alkyl, alkenyl, alkynyl, cycloalkyl, cycloalkenyl, alkylcarbonyl or alkoxycarbonyl;

$R^5$ is alkyl, alkenyl, alkynyl, cycloalkyl or cycloalkenyl, or
in the case where X is $NR^a$, additionally is hydrogen.

**2.** A process for preparing the compounds of the formula I as claimed in claim 1, where $R^4$ is hydrogen and X is a direct bond or oxygen, which comprises converting a benzyl derivative of the formula II

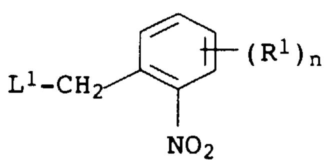

II

where $L^1$ is a nucleophilically replaceable group, in the presence of a base using a 3-hydroxy(dihydro)pyrazole of the formula III

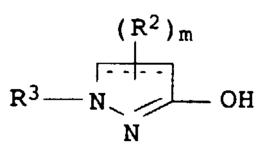

III

to the corresponding 2-[(dihydro)pyrazol-3'-yloxymethylene]nitrobenzene of the formula IV

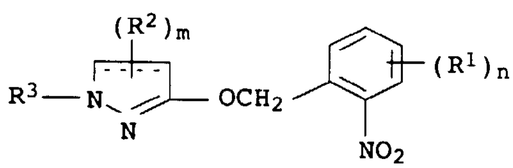

IV,

then reducing IV to the N-hydroxylaniline of the formula Va

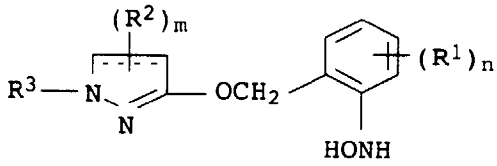

Va

and converting Va to I using a carbonyl compound of the formula VI

$L^2$-CO-X-$R^5$ VI

where $L^2$ is halogen and X is a direct bond or oxygen.

**3.** A process for preparing the compounds of the formula I as claimed in claim 1, where $R^4$ is not hydrogen and X is a direct bond or oxygen, which comprises first reducing a benzyl derivative of the formula IIa

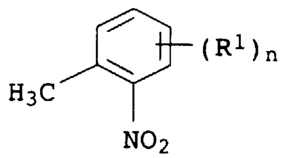

IIa

to the corresponding hydroxyaniline of the formula Vb

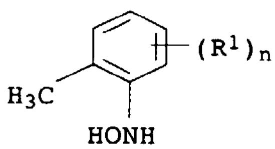

Vb,

converting Vb using a carbonyl compound of the formula VI as claimed in claim 2 to the corresponding anilide of the formula VII

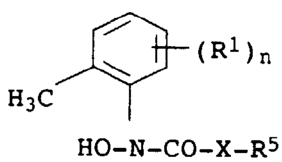

VII,

then converting VII using a compound VIII

$L^3$-$R^4$ VIII

where $L^3$ is a nucleophilically replaceable group and $R^4$ is not hydrogen, to the amide of the formula IX

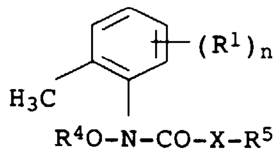

IX,

then converting IX to the corresponding benzyl halide of the formula X

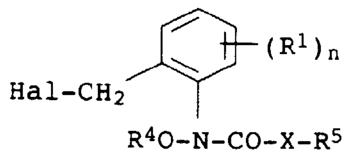

X

where Hal is a halogen atom, and converting X to I in the presence of a base using a 3-hydroxy(dihydro)pyrazole of the formula III as claimed in claim 2.

**4.** A process for preparing the compounds I as claimed in claim 1, where $R^4$ is not hydrogen and X is a direct bond or oxygen, which comprises reacting a corresponding compound of the formula I where $R^4$ is hydrogen with a compound of the formula VIII as claimed in claim 3.

**5.** A process for preparing the compounds of the formula I as claimed in claim 1, where X is $NR^a$, which comprises converting a benzylanilide of the formula IXa

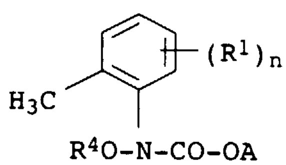

IXa

where A is alkyl or phenyl to the corresponding benzyl halide of the formula Xa

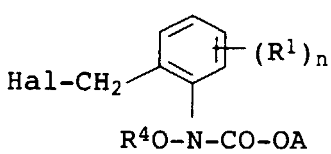

Xa

where Hal is a halogen atom, converting Xa in the presence of a base using a 3-hydroxy(dihydro)pyrazole of the formula III as in claim 2 to a compound of the formula I.A

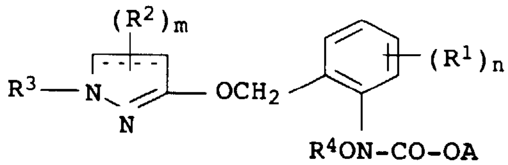

I.A

and then reacting I.A with an amine of the formula XI

$$H_2NR^a \quad (XIa)$$

$$HNR^aR^5 \quad (XIb)$$

to give I.

**6.** An intermediate of the formula XIIa

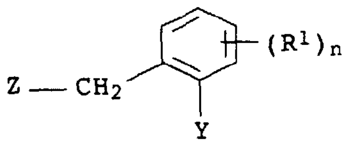

XIIa

where the substituents and the index have the following meanings:

n is 0, 1, 2, 3 or 4, it being possible for the substituents $R^1$ to be different if n is greater than 1;

$R^1$ is nitro, cyano, halogen,

unsubstituted or substituted alkyl, alkenyl, alkynyl, alkoxy, alkenyloxy, alkynyloxy or

in the case where n is 2, additionally is an unsubstituted or substituted bridge bonded to two adjacent ring atoms and containing three to four members from the group consisting of 3 or 4 carbon atoms, 1 to 3 carbon atoms and 1 or 2 nitrogen, oxygen and/or sulfur atoms, this bridge together with the ring to which it is bonded being able to form a partly unsaturated or aromatic radical;

Y is $NO_2$, NHOH or $NHOR^4$,

$R^4$ is unsubstituted or substituted alkyl, alkenyl, alkynyl, cycloalkyl, cycloalkenyl, alkylcarbonyl or alkoxycarbonyl;

$Z^a$ is a group

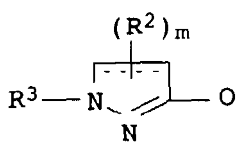

$Z^a$

m is 0, 1 or 2, it being possible for the substituents $R^2$ to be different if m is greater than 1;

$R^2$ is nitro, cyano, halogen, $C_1$-$C_4$-alkyl, $C_1$-$C_4$-haloalkyl, $C_1$-$C_4$-alkoxy, $C_1$-$C_4$-alkylthio or $C_1$-$C_4$-alkoxycarbonyl;

$R^3$ is unsubstituted or substituted alkyl, alkenyl or alkynyl;

an unsubstituted or substituted, saturated or mono- or diunsaturated ring which, in addition to carbon atoms, can contain one to three of the following heteroatoms as ring members: oxygen, sulfur and nitrogen, or

an unsubstituted or substituted, mono- or binuclear aromatic radical which, in addition to carbon atoms, can contain one to four nitrogen atoms or one or two nitrogen atoms and one oxygen or

sulfur atom or one oxygen or sulfur atom as ring members.

**7.** A composition suitable for controlling animal pests or harmful fungi, containing a solid or liquid carrier and a compound of the general formula I as claimed in claim 1.

**8.** An intermediate of the general formula XIII

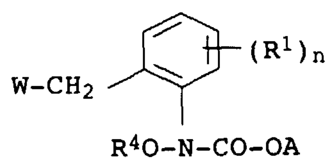

XIII

where the substituents $R^1$ and $R^4$ and also the index n have the meanings given in claim 1 and the substituents W and A have the following meanings:

W is a group $Z^a$ which has the meanings given in claim 6, and

A is phenyl.

**9.** The use of the compounds I as claimed in claim 1 for preparing a composition suitable for controlling animal pests or harmful fungi.

**10.** A method of controlling harmful fungi, which comprises treating the fungi or the materials, plants, soil or seed to be protected from fungal attack with an active amount of a compound of the general formula I as claimed in claim 1.

**11.** A method of controlling animal pests, which comprises treating the pests or the materials, plants, soil or seed to be protected from them with an active amount of a compound of the general formula I as claimed in claim 1.

**Revendications**

**1.** 2-[(dihydro)pyrazolyl-3'-oxyméthylène]anilides de formule I

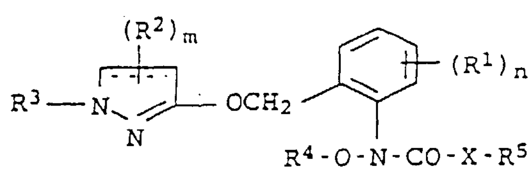

I

dans laquelle -- représente une liaison simple ou double et les indices et symboles ont les significations suivantes :

n est égal à 0, 1, 2, 3 ou 4, les symboles $R^1$ pouvant avoir des significations différentes lorsque n est supérieur à 1 ;
m est égal à 0, 1 ou 2, et les symboles $R^2$ peuvent avoir des significations identiques ou différentes lorsque n est supérieur à 1 ;
X représente une liaison directe, O ou $NR^a$ ;
$R^a$ représente l'hydrogène, un groupe alkyle, alcényle, alcynyle, cycloalkyle ou cycloalcényle ;
$R^1$ représente un groupe nitro, cyano, un halogène,

un groupe alkyle, alcényle, alcynyle, alcoxy, alcényloxy, alcynyloxy éventuellement substitué, ou bien lorsque n est égal à 2, le substituant peut consister effectivement en un pont éventuellement substitué, relié à deux atomes cycliques voisins et qui contient trois à quatre chaînons consistant en trois à quatre atomes de carbone, un à trois atomes de carbone et un ou deux atomes d'azote, d'oxygène et/ou de

soufre, ce pont pouvant former, avec le cycle auquel il est relié, un radical partiellement insaturé ou aromatique ;

$R^2$ représente un groupe nitro, cyano, un halogène, un groupe alkyle en C1-C4, halogéno-alkyle en C1-C4, alcoxy en C1-C4, alkylthio en C1-C4 ou (alcoxy en C1-C4)carbonyle ;
$R^3$ représente un groupe alkyle, alcényle ou alcynyle éventuellement substitué ;

un cycle saturé ou mono- ou di-insaturé et éventuellement substitué qui, en plus des atomes de carbone, peut contenir de un à trois des hétéroatomes suivants en tant que chaînons cycliques : l'oxygène, le le soufre et l'azote, ou bien un radical aromatique mono-ou bi-cyclique éventuellement substitué qui, en plus des atomes de carbone, peut contenir un à quatre atomes d'azote ou un ou deux atomes d'azote et un atome d'oxygène ou de soufre ou un atome d'oxygène ou de soufre en tant que chaînons cycliques ;

$R^4$ représente l'hydrogène,
un groupe alkyle, alcényle, alcynyle, cycloalkyle, cycloalcényle, alkylcarbonyle ou alcoxycarbonyle éventuellement substitué :;
$R^5$ représente un groupe alkyle, alcényle, alcynyle, cycloalkye ou cycloalcényle, ou bien
lorsque X représente $NR^a$, $R^5$ peut représenter en outre l'hydrogène.

**2.** Procédé de préparation des composés de formule I selon la revendication 1 dans laquelle $R^4$ représente l'hydrogène et X une liaison directe ou l'oxygène, caractérisé par le fait que l'on convertit un dérivé benzylique de formule II

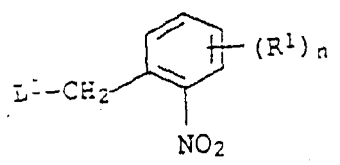

II

dans laquelle $L^1$ représente un groupe nucléophile échangeable, par réaction en présence d'une base avec un 3-hydroxy-(dihydro)pyrazole de formule III

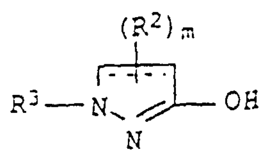

III

en le 2-[(dihydro)pyrazolyl-3'-oxyméthylène]nitrobenzène correspondant de formule IV

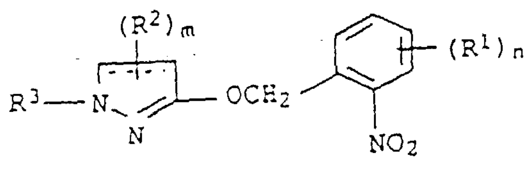

IV

qu'on convertit ensuite par réduction en une N-hydroxyaniline de formule Va

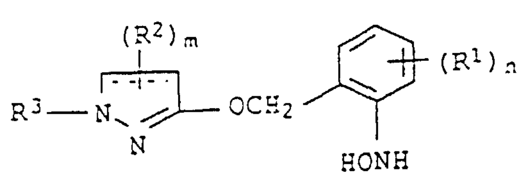

Va

qu'on convertit en I par réaction avec un dérivé carbonylé de formule VI

$$L^2\text{-CO-X-R}^5 \qquad \text{VI}$$

dans laquelle $L^2$ représente un halogène et X une liaison directe ou l'oxygène.

3. Procédé de préparation des composés de formule I de la revendication 1 dans laquelle $R^4$ a une signification autre que l'hydrogène et X représente une liaison directe ou l'oxygène, caractérisé par le fait que l'on réduit d'abord un dérivé benzylique de formule IIa

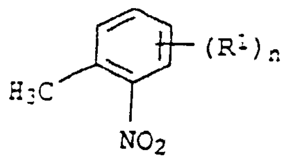

IIa

en l'hydroxyaniline correspondante de formule Vb

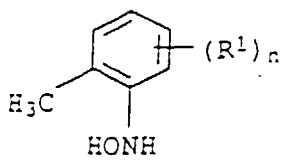

Vb

qu'on convertit par réaction avec un dérivé carbonylé de formule VI de la revendication 2 en l'anilide correspondant de formule VII

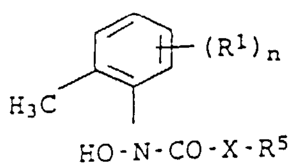

VII

qu'on convertit ensuite, par réaction avec un composé de formule VIII

$$L^3\text{-R}^4 \qquad \text{VIII}$$

dans laquelle $L^3$ représente un groupe nucléophile échangeable et $R^4$ a une signification autre que l'hydrogène, en l'amide de formule IX

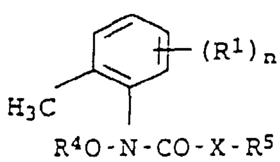

IX

qu'on convertit ensuite en l'halogénure de benzyle correspondant de formule X

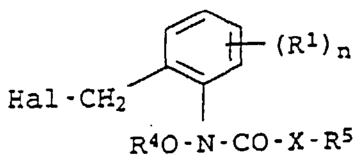

X

dans laquelle Hal représente un atome d'halogène, qu'on convertit lui-même, en présence d'une base, par réaction avec un 3-hydroxy-(dihydro)pyrazole de formule III de la revendication 2, en I.

**4.** Procédé de préparation des composés de formule I de la revendication 1 dans laquelle $R^4$ a une signification autre que l'hydrogène et X représente une liaison directe ou l'oxygène, caractérisé par le fait que l'on fait réagir un composé correspondant de formule I dans laquelle $R^4$ représente l'hydrogène avec un composé de formule VIII de la revendication 3.

**5.** Procédé de préparation des composés de formule I de la revendication 1 dans laquelle X représente $NR^a$, caractérisé par le fait que l'on convertit un benzylanilide de formule IXa.

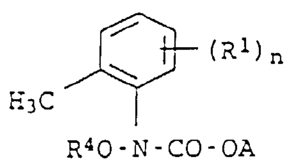

IXa

dans laquelle A représente un groupe alkyle ou phényle, en l'halogénure de benzyle correspondant de formule Xa

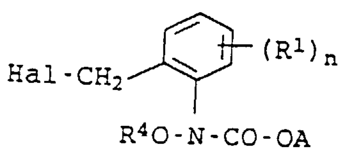

Xa

dans laquelle Hal représente un atome d'halogène, qu'on convertit ensuite en présence d'une base, par réaction avec un 3-hydroxy-(dihydro)pyrazole de formule III de la revendication 2, en un composé de formule IA

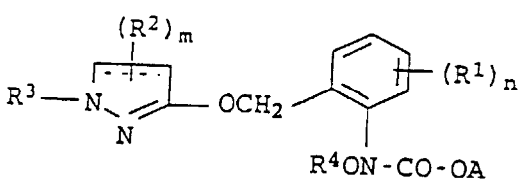

I.A

qu'on convertit ensuite par réaction avec une amine de formule XI

$$H_2NR^a \quad \text{(XIa)}$$

$$HNR^aR^5 \quad \text{(XIb)}$$

en I.

**6.** Produits intermédiaires de formule XIIa

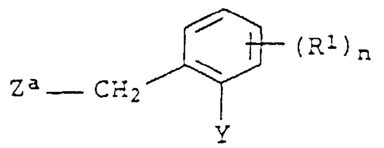

XIIa

dans laquelle les symboles et l'indice ont les significations suivantes :

n est égal à 0, 1, 2, 3, ou 4, les symboles $R^1$ pouvant avoir des significations différentes lorsque n est supérieur à 1 ;
$R^1$ représente un groupe nitro, cyano, un halogène,

un groupe alkyle, alcényle, alcynyle, alcoxy, alcényloxy, alcynyloxy éventuellement substitué, ou bien lorsque n est égal à 2, le substituant peut consister en outre en un pont éventuellement substitué relié à deux atomes cycliques voisins, qui contient trois à quatre chaînons consistant en trois ou quatre atomes de carbone, un à trois atomes de carbone et un ou deux atomes d'azote, d'oxygène et/ou de soufre, ce pont pouvant former, avec le cycle auquel il est relié, un radical partiellement insaturé ou aromatique ;

Y représente $NO_2$, NHOH ou $NHOR^4$,
$R^4$ représente un groupe alkyle, alcényle, alcynyle, cycloalkyle, cycloalcényle, alkylcarbonyle ou alcoxycarbonyle éventuellement substitué ;
$Z^a$ représente un groupe

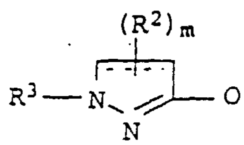

$Z^a$

dans lequel

m est égal à 0, 1 ou 2, les symboles $R^2$ pouvant avoir des significations différentes lorsque m est supérieur à 1 ;
$R^2$ représente un groupe nitro, cyano, un halogène, un groupe alkyle en C1-C4, halogéno-alkyle en C1-C4, alcoxy en C1-C4, alkylethio en C1-C4, ou (alcoxy en C1-C4)carbonyle ;
R3 représente un groupe alkyle, alcényle ou alcynyle éventuellement substitué ;

un cycle saturé ou mono- ou di-insaturé et éventuellement substitué qui, en plus des atomes de carbone, peut contenir un à trois des hétéroatomes suivants en tant que chaînons cyclique : l'oxygène, le soufre et l'azote, ou bien
un radical aromatique mono- ou bi-cyclique éventuellement substitué qui, en plus des atomes de carbone, peut contenir un à quatre atomes d'azote ou un ou deux atomes d'azote et un atome d'oxygène ou de soufre ou un atome d'oxygène ou un atome de soufre en tant que chaînons cycliques.

**7.** Produit convenant à l'utilisation pour la lutte contre les parasites animaux ou les mycètes nuisibles, contenant un véhicule solide ou liquide et un composé de formule générale I de la revendication 1.

**8.** Produits intermédiaires de formule générale XIII

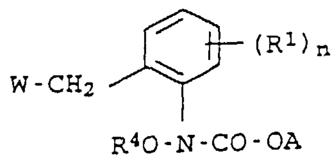

XIII

dans laquelle les symboles $R^1$ et $R^4$ et l'indice n ont les significations indiquées dans la revendication 1 et les symboles W et A ont les significations suivantes :

W représente un groupe $Z^a$; à la signification indiquée dans la revendication 6 et
A représente un groupe phényle.

**9.** Utilisation des composés I selon la revendication 1 pour la préparation d'un produit convenant à l'utilisation pour la lutte contre les parasites animaux ou mycètes nuisibles.

**10.** Procédé pour combattre les mycètes nuisibles, caractérisé par le fait que l'on traite les mycètes ou les matériaux, végétaux, sols ou semences à protéger contre l'attaque par les mycètes, par une quantité efficace d'un composé de formule générale I de la revendication 1.

**11.** Procédé pour combattre les parasites animaux, caractérisé par le fait que l'on traite les parasites ou les matériaux, végétaux, sols ou semences à protéger contre les parasites par une quantité efficace d'un composé de formule générale I de la revendication 1.